# EUROPEAN PATENT SPECIFICATION

(11) **EP 2 298 883 B1**
(45) Date of publication and mention of the grant of the patent: **27.07.2016**
(21) Application number: 09754688.1
(22) Date of filing: 26.05.2009
(51) Int. Cl.: C12N 15/82

(54) **NOVEL SELECTION MARKER GENE AND USE THEREOF**
NEUES SELEKTIONSMARKERGEN UND VERWENDUNG DAVON
NOUVEAU GÈNE MARQUEUR DE SÉLECTION ET SON UTILISATION

(30) Priority: 28.05.2008 JP 2008140083
(43) Date of publication of application: 23.03.2011
(73) Proprietor: Kyoto University, Kyoto-shi Kyoto 606-8501 (JP)
(72) Inventor: NISHIMURA, Ikuko, Kyoto 606-8502 (JP); SHIMADA, Tomoo, Kyoto 606-8502 (JP); SHIMADA, Takashi, Kyoto 606-8502 (JP)
(74) Representative: Cabinet Plasseraud
(86) International application number: PCT/JP2009/059592
(87) International publication number: WO 2009/145180

(56) References cited:
- WO-A1-99/60129
- JP-T- 2007 510 420
- HUI LIU ET AL: "Characterisation and functional analysis of two barley caleosins expressed during barley caryopsis development", PLANTA ; AN INTERNATIONAL JOURNAL OF PLANT BIOLOGY, SPRINGER, BERLIN, DE, vol. 221, no. 4, 1 June 2005 (2005-06-01), pages 513-522, XP019344285, ISSN: 1432-2048, DOI: 10.1007/S00425-004-1465-5
- DE DOMENICO STEFANIA ET AL: "Subcellular localisation of Medicago truncatula 9/13-hydroperoxide lyase reveals a new localisation pattern and activation mechanism for CYP74C enzymes", BMC PLANT BIOLOGY, vol. 7, November 2007 (2007-11), XP0021033818, ISSN: 1471-2229, DOI: 10.1186/1471-2229-7-58
- PENG CHI-CHUNG ET AL: "A system for purification of recombinant proteins in Escherichia coli via artificial oil bodies constituted with their oleosin-fused polypeptides", JOURNAL OF BIOTECHNOLOGY, vol. 111, no. 1, 1 July 2004 (2004-07-01), pages 51-57, XP000002659232, ISSN: 0168-1656
- HUA LING: "Oleosin fusion expression systems for the production of recombinant proteins", BIOLOGIA, vol. 62, no. 2, 1 April 2007 (2007-04-01), pages 119-123, XP55007378, ISSN: 0006-3088, DOI: 10.2478/s11756-007-0041-4
- MATHUR ET AL: "The illuminated plant cell", TRENDS IN PLANT SCIENCE, ELSEVIER SCIENCE, OXFORD, GB, vol. 12, no. 11, 22 October 2007 (2007-10-22), pages 506-513, XP022324928, ISSN: 1360-1385, DOI: 10.1016/J.TPLANTS.2007.08.017
- WAHLROOS T ET AL.: 'Oleosin expression and trafficking during oil body biogenesis in tobacco leaf cells' GENESIS. vol. 35, no. 2, 2003, pages 125 - 132, XP008141026
- 'Saibonai Kozo no Kochiku to Kino no Kansatsuho, Oil Body no Kozo to Kino, Saibo Kogaku Bessatsu Shokubutsu Saibo Kogaku Series' NEW EDITION SHOKUBUTSU NO SAIBO O MIRU JIKKEN PROTOCOL vol. 22, no. 2ND ED, 2006, pages 219, 220 - 222, XP008142975
- CURTIS I.S. ET AL.: 'Transgenic radish (Raphanus sativus L. longipinnatus Bailey) by floral-dip method--plant development and surfactant are important in optimizing transformation efficiency.' TRANSGENIC RES. vol. 10, no. 4, 2001, pages 363 - 371, XP002416814

## Description

### Technical Field

The present invention relates to a novel selection marker gene and use thereof. To be more specific, the present invention relates to a gene which encodes a fusion protein of a seed protein and a fluorescent protein and use of the gene.

### Background Art

A drug-resistance gene is generally used as a selection marker when preparing a transformant. However, the technique of preparing a transformant using a drug-resistance gene is problematic in terms of the following points.
[1] There is a possibility that a plant's gene is horizontally transferred when the plant is cultivated. Accordingly, cultivation of a plant having a drug-resistance gene outside is restricted.
[2] A treatment with drug is required when selecting a desired transformant. Accordingly, it is necessary to separately prepare a drug-containing culture medium used in the selection.
[3] Even a plant having a drug-resistance gene is damaged by the treatment with drug.
[4] It is difficult to obtain a transformant which is too weak to be cultured in a drug-containing culture medium.

In order to solve these problems, preparation of a transgenic plant having no drug-resistance gene has been tried (see Non-patent Literatures 1 and 2 for example). Further, in a process called co-transformation, two plasmids (one includes a drug-resistance gene marker and the other includes a target transgenic gene) are simultaneously introduced into a plant and after several generations, it is possible to select a plant which does not have a drug-resistance gene but has a desired transforming gene (see Non-patent Literature 3 for example). Further, a process for removing a drug-resistance gene marker from a transgenic plant with use of a site-specific recombination mechanism (site-specific recombination) has been known (see Non-patent Literatures 4-7 for example).

### Citation List

### [Non-patent Literatures]

[Non-patent Literature 1]
   John I. Yoder, A.P.G. Nature Biotechnology 12, 263 - 267 (1994)
[Non-patent Literature 2]
   Darbani et al., Biotechnol. J. 2, 83-90 (2007)
[Non-patent Literature 3]
   Parkhi, V. et al., Mol. Genet. Genomics 274, 325-336 (2005)
[Non-patent Literature 4]
   Zuo, J. et al., Nat. Biotechnol. 19, 157-161 (2001)
[Non-patent Literature 5]
   Li, Z. et al., Plant Mol. Biol. 65, 329-341 (2007)
[Non-patent Literature 6]
   Hu, Q. et al., Biotechnol. Lett. 28, 1793-1804 (2006)
[Non-patent Literature 7]
   Sugita, K. et al., Plant J. 22, 461-469 (2000)
[Non-patent Literature 8]
   Baranski, R. et al., Plant Cell Rep 25, 190-197 (2006)
[Non-patent Literature 9]
   Halfhill, M.D. et al., Plant Cell Rep. 26, 303-311 (2007)
[Non-patent Literature 10]
   Lu, C. et al., Plant J. 45, 847-856 (2006)
[Non-patent Literature 11]
   Lu, C. and Kang, J. Plant Cell Rep. 27, 273-278 (2008)

### Summary of Invention

### Technical Problem

Use of co-transformation or site-specific recombination allows preparing a transgenic plant having no drug-resistance gene. However, co-transformation and site-specific recombination require a complicated process, and require a time to prepare a transgenic plant.

The present invention was made in view of the foregoing problems. An object of the present invention is to provide a technique of obtaining a desired transformant in a relatively short time without requiring a complicated process, for the purpose of preparing a transgenic plant.

### Solution to Problem

A process of using a selection marker other than a drug-resistance gene for selecting a transgenic plant has been known, too. A green fluorescent protein (GFP), which is one of fluorescent proteins used as a visual selection marker, is innocuous to an organism, and can be made visible easily without using a substrate (see Non-patent Literatures 8-9 for example). Further, a transgenic seed selection marker using a fluorescent protein other than GFP has been known (see

### Non-patent Literatures 10-11 for example).

While studying a seed protein, the inventors of the present invention have found that a gene which is operably linked to a seed-specific promoter and which encodes a fusion protein of a seed protein and a fluorescent protein is not only excellent as a visual selection marker but also usable as a codominant marker, and thus completed the present invention.

That is, a DNA construct of the present invention includes a gene encoding a fusion protein of a seed protein and a fluorescent protein, the gene being operably linked to a seed-specific promoter.

The present invention relates to a DNA construct, a transgenic plant, a method for selecting a transgenic seed and a method for producing a protein in a plant, as defined in the claims. In particular, the seed protein used in the present invention is OLE1, consisting of an amino acid sequence represented by SEQ ID NO:1, and the seed-specific promoter used in the present invention is an oleosin promoter consisting of a base sequence represented by SEQ ID NO: 6.

With the present invention, it is possible to easily select a successively transformed individual as a seed with detectable fluorescence. Fluorescence detected from a seed with use of the present invention is extremely stronger than fluorescence from a seed obtained when only a gene encoding a fluorescent protein is operably linked to a seed-specific promoter. Further, with the present invention, it is possible to obtain a transgenic plant whose seed expresses a fluorescent protein but whose seedling resulting from the seed (e.g. root, leaf, and shaft) does not express a fluorescent protein. In contrast thereto, in the techniques described in Non-patent Literatures 8-11, a fluorescent protein is expressed in all tissues, since expression of the fluorescent protein is controlled by a strong promoter (CaMV 35S promoter or pCVMV promoter).

The DNA construct of the present invention may be arranged such that a second gene encoding a target protein and a gene encoding a second fluorescent protein are operably linked to the seed-specific promoter, and the second fluorescent protein is a protein that emits fluorescence with a color different from a color of fluorescence of the fluorescent protein constituting the fusion protein of the seed protein and the fluorescent protein.

With the arrangement, it is possible to visually distinguish fluorescence emitted from the second fluorescent protein from fluorescence emitted from the fluorescent protein constituting the fusion protein. Accordingly, it is possible to visually separately detect expression of the fusion protein and expression of the target protein.

It is possible not only to easily select a transformed individual as a seed with detectable fluorescence but also to detect expression of the target protein in a seed distinctly from expression of a selection marker.

The DNA construct of the present invention may be arranged so as to further include a second promoter for expressing a target protein in a target tissue, and a gene encoding the target protein is operably linked to the second promoter.

The techniques described in Non-patent Literatures 10 and 11 are techniques for accumulating a target protein in a seed, and a gene encoding the target protein is operably linked to a seed-specific promoter. In contrast thereto, with the present invention having the aforementioned arrangement, it is possible to express a target gene in a target tissue which is not limited to a seed, thereby accumulating a target protein in the target tissue. Also in this case, in the resulting transformant, only a seed expresses a fluorescent protein and seedling resulting from the seed (e.g. root, leaf, and shaft) does not express the fluorescent protein. Further, expression of the fluorescent protein and expression of the target protein do not interfere with each other.

The DNA construct of the present invention may be arranged such that a second gene encoding a target protein and a gene encoding a second fluorescent protein are operably linked to the second promoter, and the second fluorescent protein is a protein that emits fluorescence with a color different from a color of fluorescence of the fluorescent protein constituting the fusion protein of the seed protein and the fluorescent protein.

With the arrangement, the fluorescent protein expressed in a seed emits fluorescence with a color different from that of fluorescence of the fluorescent protein expressed in the target tissue (second fluorescent protein) emit fluorescence with different colors, and expression of the fluorescent protein expressed in a seed and expression of the second fluorescent protein do not interfere with each other. Accordingly, it is possible not only to easily select a transformed individual as a seed with detectable fluorescence but also to easily confirm expression of the target protein in the target tissue.

In the DNA construct of the present invention, the seed protein is preferably an oil body-localized protein, and the oil body-localized protein is more preferably a protein selected from the group consisting of oleosin, caleosin, and steroleosin. Further, in the DNA construct of the present invention, the seed-specific promoter is a native promoter of a gene encoding the oil body-localized protein. When this promoter is used, it is extremely easier to detect fluorescence from a seed than when a promoter directed to other organelle in a seed, and therefore the DNA construct of the present invention is extremely superior as a visual selection marker at a seed stage. A promoter of a gene encoding the oil body-localized protein is more preferably a promoter of a gene encoding protein selected from a group consisting of oleosin, caleosin, and steroleosin.

In the DNA construct of the present invention, the fusion protein is preferably made by fusing a fluorescent protein with a C-terminus of a seed protein.

A selection marker of the present invention includes the DNA construct. Further, a selection marker kit of the present invention includes the DNA construct.

A transgenic plant of the present invention is a transgenic plant, to which a gene encoding a fusion protein of a seed protein and a fluorescent protein is introduced, the gene being operably linked to a seed-specific promoter. The transgenic plant of the present invention preferably may be at least one of a grown-up plant, a plant cell, a plant tissue, a callus, and a seed.

A method of the present invention for selecting a transgenic plant includes the step of detecting that a gene encoding a fusion protein of a seed protein and a fluorescent protein exists in a seed, the gene being operably linked to a seed-specific promoter. In the method, the step of detecting may include detecting fluorescence of the fluorescent protein from a seed or may include detecting a gene encoding the fusion protein or a gene encoding the fluorescent protein from a seed extract.

The method of the present invention may be arranged so as to further include the step of detecting that a gene which is operably linked to a seed-specific promoter and which encodes a second fluorescent protein exists in a seed.

The method of the present invention may be arranged so as to further include the step of detecting that a gene which is operably linked to a second promoter and which encodes a second fluorescent protein exists in a target tissue.

A method of the present invention for producing a protein in a plant includes the steps of: (1) inserting, to a DNA construct including a gene which is operably linked to a seed-specific promoter and which encodes a fusion protein of a seed protein and a fluorescent protein, a second gene encoding a target protein; and (2) introducing the DNA construct obtained in the step (1) to a plant. It is preferable to arrange the method of the present invention such that the DNA construct further includes a second promoter for expressing a target protein in a target tissue, and the step (1) includes operably linking the second gene to the second promoter. Further, it is preferable to arrange the method of the present invention such that the step (2) includes carrying out floral-dip or vacuum infiltration.

For a fuller understanding of other objects, nature and advantages of the invention, reference should be made to the ensuing detailed description taken in conjunction with the accompanying drawings.

### Advantageous Effects of Invention

Use of the present invention allows more easily and efficiently selecting a transgenic plant than when a drug-resistance marker is used. Further, the present invention is usable as a codominant maker which is capable of easily distinguishing a homogeneous series from a hetero series.

### Brief Description of Drawings

Fig. 1

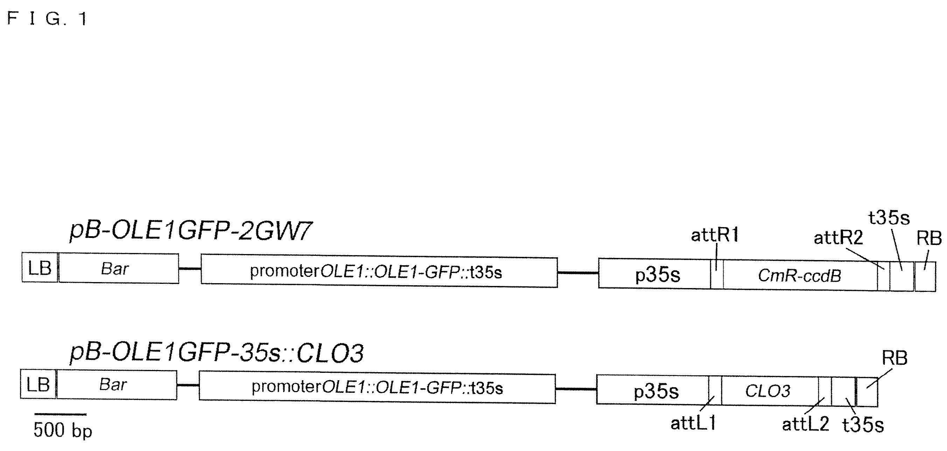

   Fig. 1 is a drawing showing a structure of a DNA construct of the present invention.
Fig. 2

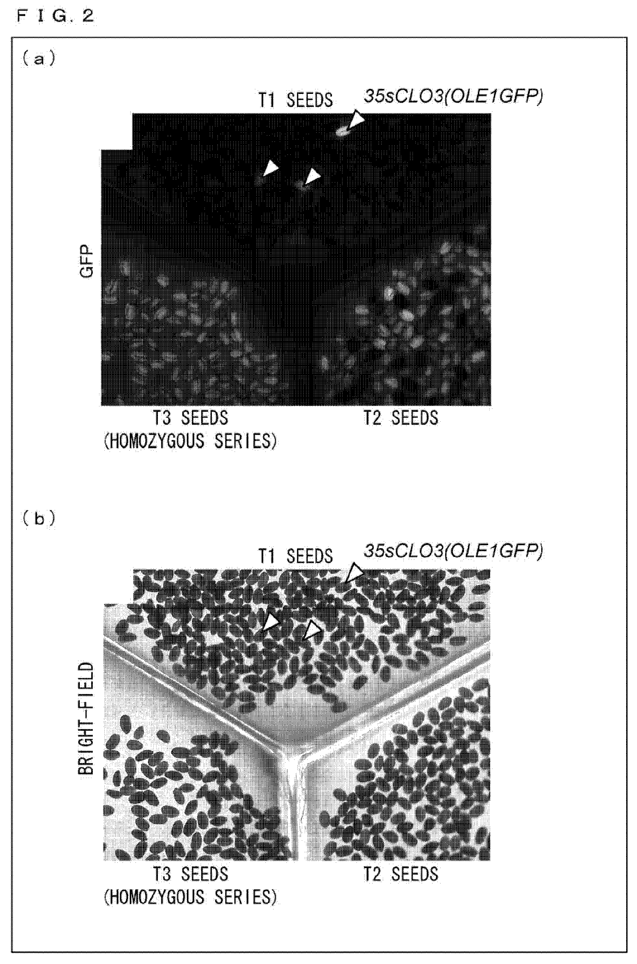

   Fig. 2 is a drawing showing the result of observation with a fluorescent microscope of seeds of a plant series to which a vector for excessively expressing CLO3 in accordance with one embodiment is introduced. (a) indicates GFP fluorescence, and (b) indicates a bright-field image.
Fig. 3

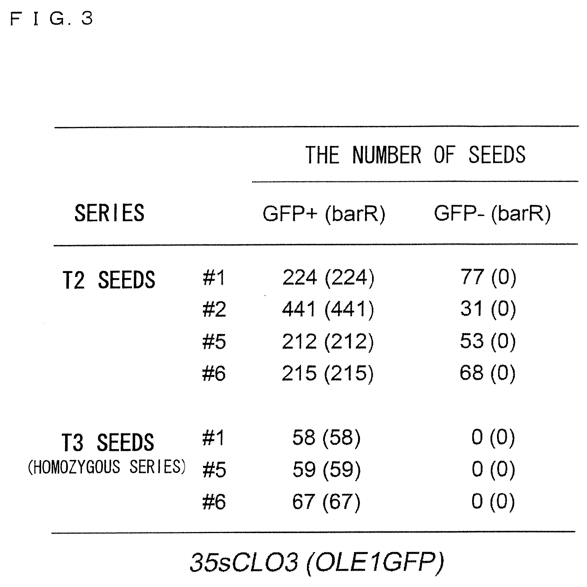

   Fig. 3 is a drawing showing: a segregation ratio of a T2 seed group and a T3 homozygous series seed group of a plant (35SCL03(OLE1GFP)) in accordance with one embodiment; and the number of seeds resistant to a drug (Glufosinate-ammonium).
Fig. 4

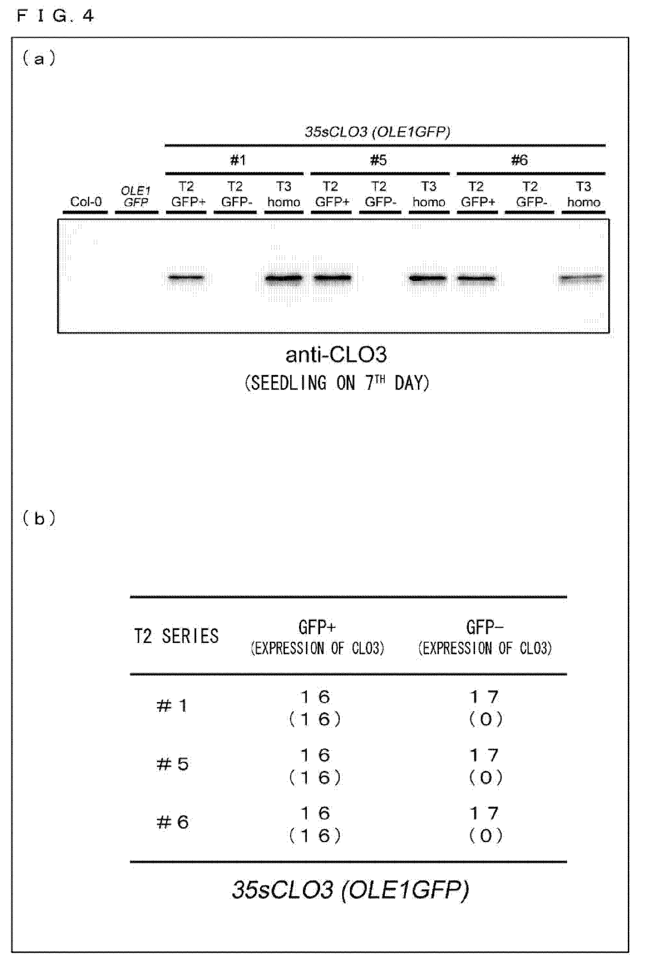

   Fig. 4 is a drawing showing the result of confirming expression of CLO3 in seeds with observed GFP fluorescence in a seed group of a plant (35SCLO3 (OLE1GFP)) in accordance with one embodiment. (a) indicates the result of examining expression of CLO3 by immunoblotting. (b) indicates the number of seeds whose expression of CLO3 was confirmed.
Fig. 5

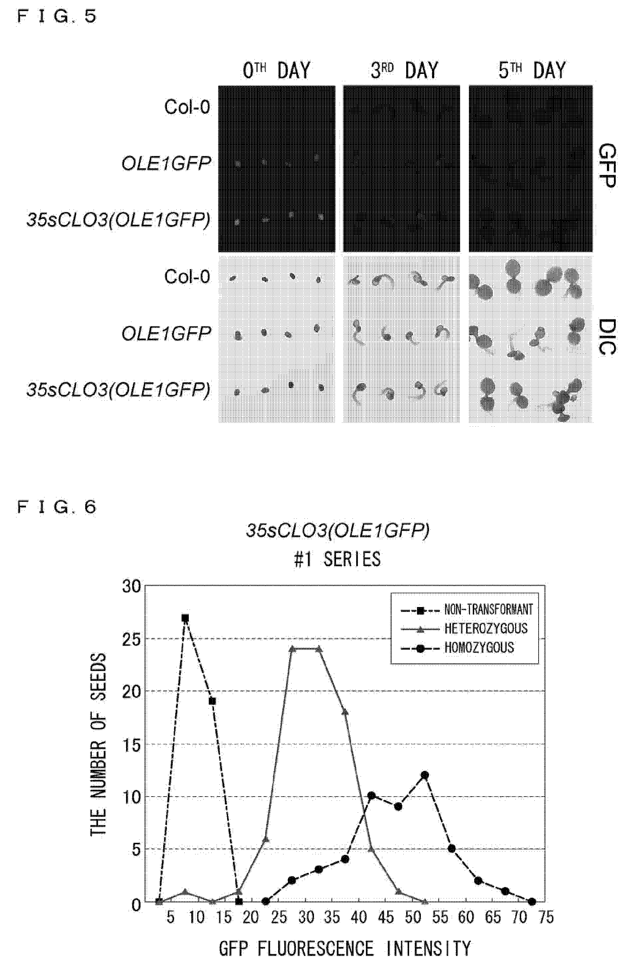

   Fig. 5 is a drawing showing transition of fluorescence in germinated OLE1GFP.
Fig. 6
   Fig. 6 is a drawing showing a relation between GFP fluorescence intensity in T2 seeds of a 35SCLO3 (OLE1GFP) plant and a genetic type of a transgenic gene.
Fig. 7

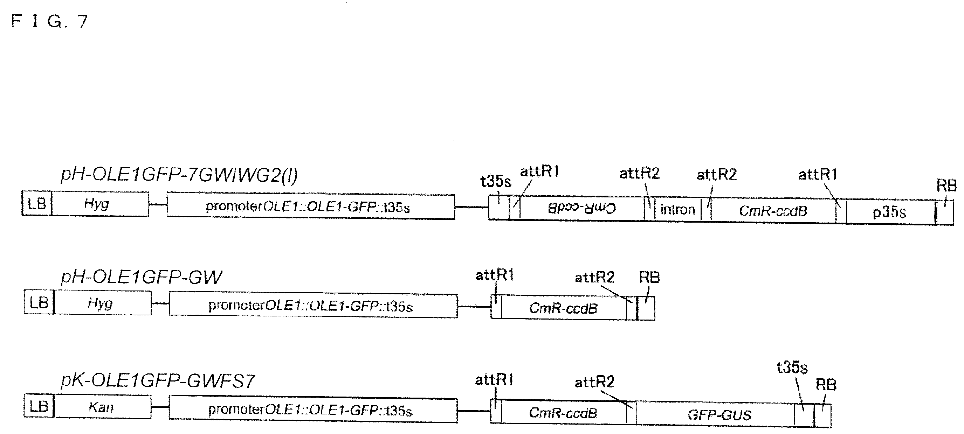

   Fig. 7 is a drawing showing a structure of a DNA construct of the present invention.
Fig. 8

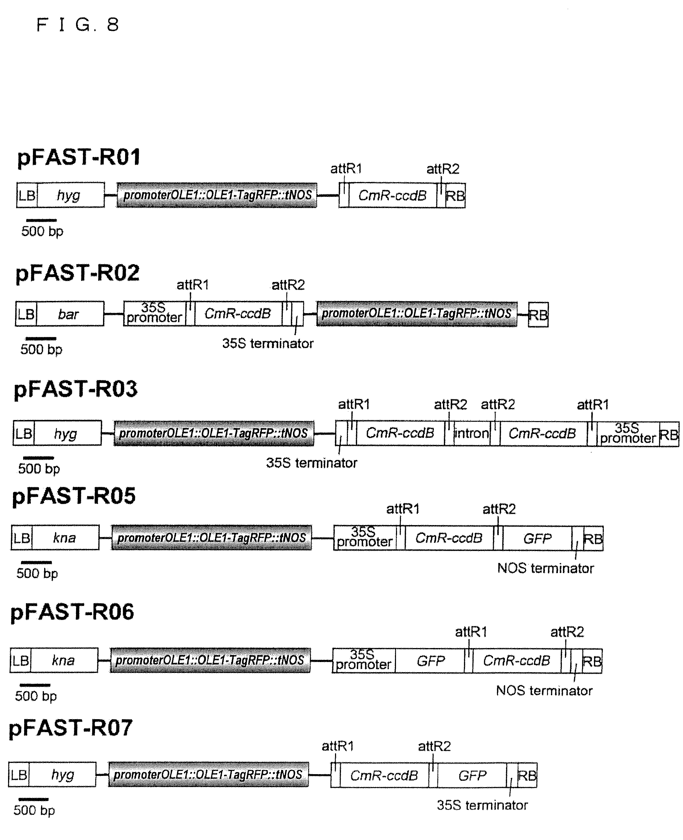

   Fig. 8 is a drawing showing a structure of a DNA construct of the present invention.
Fig. 9

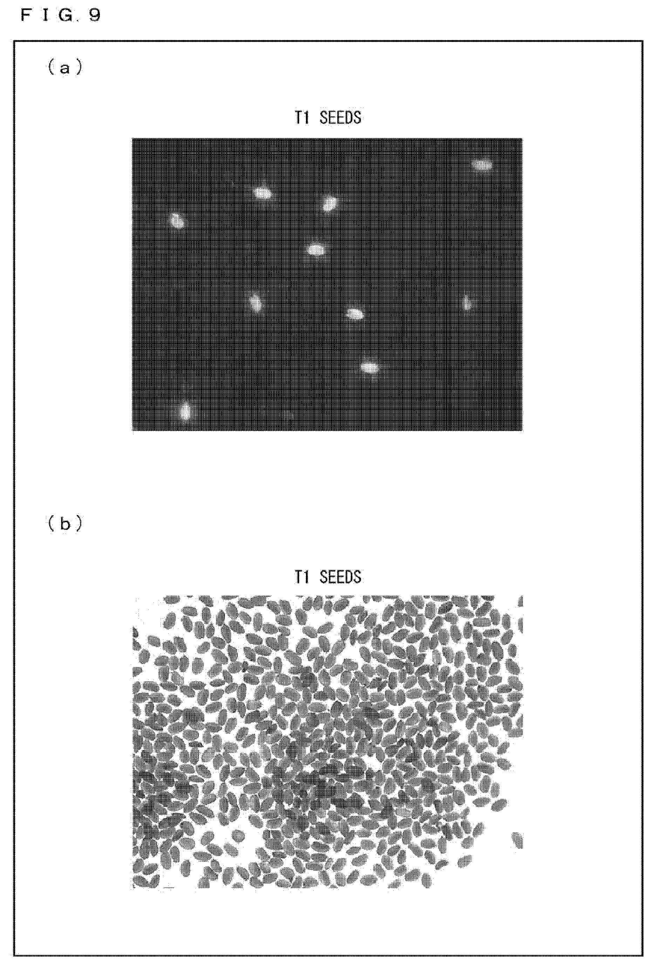

   Fig. 9 is a drawing showing the result of observation with a fluorescent microscope of a T1 seed group of a plant series to which a vector for excessively expressing a target gene under the control of 35S promoter is introduced. (a) indicates RFP fluorescence, and (b) indicates a bright-field image.
Fig. 10

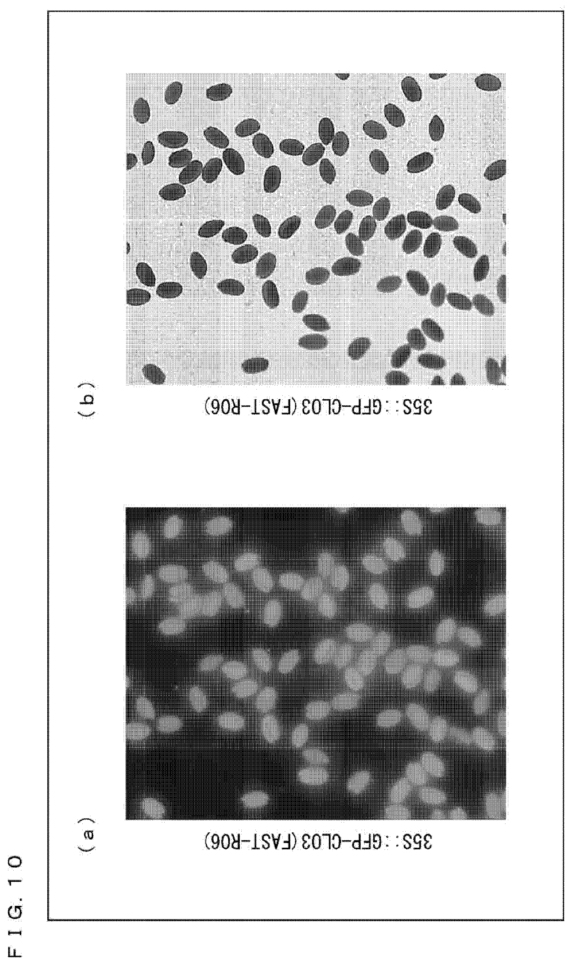

   Fig. 10 is a drawing showing the result of observation with a fluorescent microscope of T3 homozygous series seed group obtained from 355:: GFP-CLO3 (FAST-R06). (a) indicates fluorescence of TagRFP, and (b) indicates a bright-field image.
Fig. 11

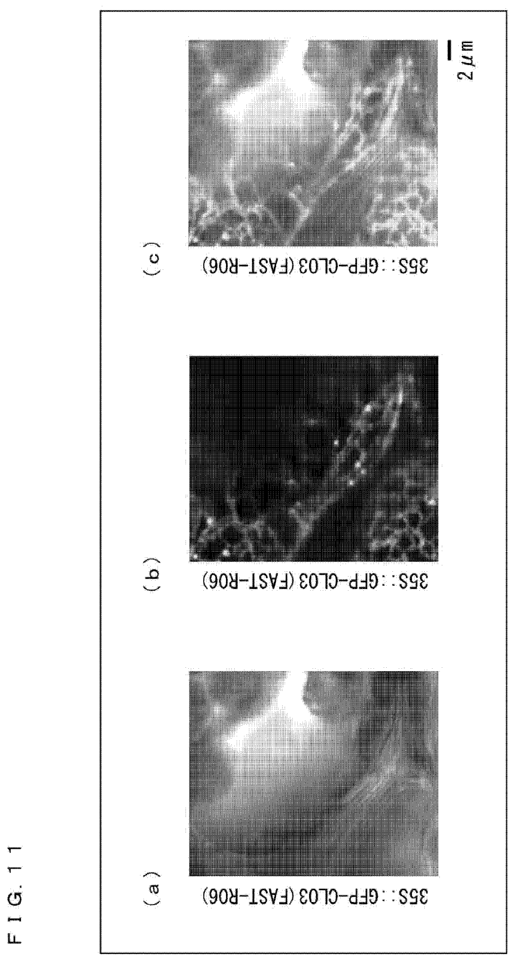

   Fig. 11 is a drawing showing the result of observing expression of CLO3 in a leaf of 35S:: GFP-CLO3(FAST-R06) with GFP fluorescence as an indicator. (a) is an image showing the result of observing the leaf with a differential interference microscope, and (b) is an image showing the result of observing the leaf with a confocal laser microscope and detecting GFP fluorescence. (c) is an image obtained by overlapping the images of (a) and (b).

### Description of Embodiments

A seed cell of a plant has an organelle for accumulating a reserve substance. Arabidopsis thaliana, which is one of oil seed plants, accumulates a large amount of reserve fat (mainly triacylglycerol) in its organelle called an oil body for accumulating a reserve substance. In the oil body, membrane proteins such as oleosin, caleosin, and steroleosin are localized. In particular, the amount of accumulated oleosin is largest among proteins localized in the oil body.

A seed oleosin is a protein accumulated in large amounts only in an oil body of a seed. A seed of Arabidopsis thaliana has main isoforms of oleosin (OLE1-4). The inventors of the present invention have prepared a transformant into which an OLE1GFP fusion gene was introduced with use of an OLE1 promoter, and observed with a fluorescent microscope that fluorescence of GFP is seen only in seeds. That is, the inventors of the present invention have found that OLE1GFP is not only usable as a transformation marker for Arabidopsis thaliana but also more useful than a conventional drug-resistance marker. Further, in a T1 seed group of a transformant to which an OLE1GFP fusion gene was introduced by floral-dip using Agrobacterium, selection of a transformant was possible with fluorescence of GFP in a seed as an indicator. Further, in a T2 seed group, seeds of a T2 homozygous series could be efficiently selected as seeds with strong fluorescence of GFP. This indicates that the OLE1GFP fusion gene is usable as a codominant marker capable of easily distinguishing a homozygous series and a heterozygous series. Selection with use of a conventional drug-resistance marker suffers from serious problems such as a possibility of horizontal gene transfer of a drug-resistance gene, necessity to prepare a selective culture medium containing a drug, and an adverse effect of the drug on a plant. In contrast thereto, selection with use of an OLE1GFP marker can be made only by observing GFP with a fluorescent microscope. This shows that selection of a transgenic plant can be made more easily and efficiently with use of the OLE1GFP marker than with use of the drug-resistance marker.

### [1] DNA construct and selection marker

The present invention provides a DNA construct usable as a novel selection marker gene. The DNA construct of the present invention includes a gene which encodes a fusion protein of a seed protein and a fluorescent protein, and the gene is operably linked to a seed-specific promoter.

A fluorescent protein has been already used as a selection marker in substitution for a drug-resistance marker. The present invention uses a fusion protein of a seed protein and a fluorescent protein, thereby providing a superior technique compared to a conventional selection with use of only a fluorescent protein.

In the specification, the wording "operably linked to" indicates that a gene for encoding a desired protein is under the control of a control region such as a promoter and is capable of expressing the protein (or mRNA) in a host. A procedure for causing a gene encoding a desired peptide to be "operably linked" to a control region such as a promoter so as to construct a desired vector is well known in the art. Further, the technique of introducing an expression vector into a host is also well known in the art. accordingly, a person skilled in the art can easily produce a desired protein (or mRNA) in a host.

A seed protein usable in the present invention may be any seed protein as long as the seed protein is specifically expressed in a seed or the seed protein is specifically expressed in an organelle in a seed. As described above, the term "seed protein" in the specification indicates not only a protein accumulated in a seed but also a protein localized in an oil body. Preferable examples of the protein accumulated in a seed include, but not limited to, 12S globulin, cucurbithin, glutelin, glycinin, legumin, arachin, conglycinin, 7S globulin, phaseolin, vicilin, conarachin, 2S globulin, amandin, prolamin, zein, gliadin, edestin, glutenin, lysine, hemagglutinin, 2S albumin, canavalin, concanavalin, trypsin inhibitor, and cystatin. Further, preferable examples of the protein localized in an oil body include oleosin (e.g. OLE1 (at4g25140)), caleosin (e.g. CL03 (at2g33380)), and steroleosin (e.g. STE1 (at5g50600)). A most preferable example of the protein localized in an oil body is oleosin (OLE1-4). Amino acid sequences of OLE1-4 are shown in SEQ ID NO: 1-4, and an amino acid sequence of CLO3 is shown in SEQ ID NO: 5. Oleosin, caleosin, and steroleosin have various isoforms and orthologs in a plant, and use of any of such isoforms and orthologs yields the effect of the present invention.

In the present invention, the seed protein may be preferably a protein accumulated in a seed or a protein localized in an oil body, and more preferably a protein localized in an oil body. In the present invention, when the protein accumulated in a seed is used, it is very difficult to observe fluorescence with a general fluorescent microscope, which requires use of a modified fluorescent protein having higher fluorescence intensity or use of a confocal laser microscope. However, when the protein localized in an oil body is used, it is possible to easily observe fluorescence from a seed with a general fluorescent microscope. The fluorescent protein usable in the present invention may be a fluorescent protein well known in the art, but GFP, RFP etc. is preferable in terms of handleability and availability.

Any of the proteins described in the specification should not be defined by a single amino acid sequence. For example, OLE1 is made of an amino acid sequence shown in SEQ ID NO: 1, and a person skilled in the art who reads the specification would easily understand that a variant of OLE1 which variant has the same function as that of OLE1 is also encompassed in the scope of OLE1. The "variant" of OLE1 used herein indicates a protein made of an amino acid sequence derived from the amino acid sequence shown in SEQ ID NO: 1 by deletion, addition, or substitution of one or several amino acids. That is, a person skilled in the art would easily understand that a protein derived from an original protein by deletion, addition, or substitution of one or several amino acids may be considered as the original protein as long as the protein maintains the function of the original protein. A person skilled in the art would easily understand the function of the original protein from the name of the original protein.

The fluorescent protein may be fused with any of an N-terminus or a C-terminus of the seed protein. When a functional site of the seed protein is located at the N-terminus, it is preferable that the fluorescent protein is fused with the C-terminus of the seed protein.

The seed-specific promoter usable in the present invention is only required to be a promoter which natively controls a gene encoding a protein specifically expressed in a seed. A preferable example of the seed-specific promoter is, but not limited to, a promoter which natively controls a gene encoding the protein accumulated in a seed, the protein localized in an oil body etc.

In the present invention, preferable examples of the seed-specific promoter include a promoter which natively controls a gene encoding a protein accumulated in a seed and a promoter which natively controls a gene encoding a protein localized in an oil body. Examples of the promoter which controls a gene encoding a protein accumulated in a seed include, but not limited to, 2S albumin 3 promoter (SEQ ID NO: 7), 12S globulin promoter (SEQ ID NO: 8), and β-conglycinin promoter (SEQ ID NO: 9). A more preferable example of the seed-specific promoter is a promoter which natively controls a gene encoding a protein localized in an oil body. As described above, in the present invention, the protein localized in an oil body exhibits a far more excellent effect as a seed protein than the protein accumulated in a seed. Accordingly, it is more preferable that the promoter which (natively) controls a gene encoding the protein localized in an oil body (e.g. oleosin promoter (proOLE1): SEQ ID NO: 6) is used as the seed-specific promoter. It should be noted that base sequences of some of the aforementioned promoters which natively control a gene encoding a seed protein (protein accumulated in a seed and protein localized in an oil body) are not demonstrated but a person skilled in the art could easily demonstrate the undemonstrated base sequences.

In one embodiment, a DNA construct of the present invention includes a gene encoding a fusion protein of a seed protein and a fluorescent protein, the gene is operably linked to a seed-specific promoter, a second gene encoding a target protein and a gene encoding a second fluorescent protein are operably linked to the seed-specific promoter, and the second fluorescent protein is a protein that emits fluorescence with a color different from a color of fluorescence of the fluorescent protein constituting the fusion protein of the seed protein and the fluorescent protein.

The second fluorescent protein is not particularly limited as long as the second fluorescent protein emits fluorescence with a color different from that of fluorescence of the fluorescent protein constituting the fusion protein, and the second fluorescent protein may be a publicly well known fluorescent protein. A protein that emits fluorescence with a color different from that of fluorescence of the fluorescent protein constituting the fusion protein may be selected from, for example, fluorescent proteins such as GFP, YFP, CFP, and RFP and other than the fluorescent protein constituting the fusion protein. The second fluorescent protein may be linked to any one of an N-terminus and a C-terminus of a target protein.

The wording "emit fluorescence with a color different from" used herein indicates that fluorescence with a wavelength in a visible light range (380-780 nm) presents human eyes with different color senses depending on a wavelength. For example, fluorescence with green-blue color(480-490 nm) is different from fluorescence with blue-green color(490-500 nm), and fluorescence with blue-green color is different from fluorescence with a green color (500-560 nm).

When the second fluorescent protein emits fluorescence with a color different from that of fluorescence of the fluorescent protein constituting the fusion protein, it is possible to detect a gene encoding the second fluorescent protein in a seed distinctly from a gene encoding the fusion protein in the seed. That is, it is possible to distinctly detect expression of a target protein in a seed distinctly from a selection marker, allowing more easily selecting a seed in which the target protein is expressed.

For example, in a case where the fluorescent protein constituting the fusion protein is RFP which emits red fluorescence, GFP which emits green fluorescence is used as the second fluorescent protein and a target protein is detected with the green fluorescence as an indicator. This allows clearly detecting expression of the target protein distinctly from a selection marker. Confirmation of the fluorescence may be made by a conventional and publicly known method, e.g. with a fluorescent microscope.

A procedure for constructing a desired vector by causing the second gene and a gene encoding the second fluorescent protein to be operably linked to the seed-specific promoter is well known in the art. Further, a method for introducing an expression vector into a host is also well known in the art.

Therefore, reading the specification, a person skilled in the art could appropriately construct an expression vector and to observe fluorescence from the fluorescent protein constituting the fusion protein and fluorescence from the second fluorescent protein in such a manner that the two fluorescence are distinguishable from each other. For example, by constructing a vector such as pFAST-R07 which is a modified destination vector constructed in a later-mentioned Example, it is possible to make observation as above.

In one embodiment, the DNA construct of the present invention further includes a second promoter for expressing a target protein in a target tissue. Since the second promoter is intended for expressing a target protein in a target tissue, any promoter publicly known in the art can be used as the second promoter. Examples of the promoter publicly known in the art include, but not limited to, 35S promoter (SEQ ID NO: 10), dexamethasone inducible promoter, estrogen-depending promoter, CHS-A promoter, heat shock promoter, RuBisCO promoter, and stress-responsive promoter. When the DNA construct of the present invention is used, surprisingly, expression of a fluorescent protein and expression of a target protein do not interfere with each other at all. For example, when a promoter other than a seed-specific promoter is used as the second promoter, only a seed in the resulting transformant expresses a fluorescent protein and seedling resulting from the seed (e.g. root, leaf, and shaft) in the transformant does not express the fluorescent protein.

In one embodiment, a second gene encoding a target protein and a gene encoding a second fluorescent protein are operably linked to the second promoter, and the second fluorescent protein is a protein that emits fluorescence with a color different from a color of fluorescence of the fluorescent protein constituting the fusion protein of the seed protein and the fluorescent protein. The second fluorescent protein may be linked to any one of an N-terminus and a C-terminus of the target protein.

By using the DNA construct of the present invention, it is possible to detect expression of a target protein in a desired tissue clearly distinctly from expression of a selection marker in a seed.

Further, it is reported that when a gene encoding the same fluorescent protein as the fluorescent protein constituting the fusion protein instead of a gene encoding the second fluorescent protein is operably linked to the second promoter, expression of a target protein tends to be suppressed (C. B. Taylor, Comprehending Cosuppression, Plant Cell 9: 1245-1249.,1997). In contrast thereto, in the present embodiment, since a gene encoding the second fluorescent protein different from the fluorescent protein constituting the fusion protein is used, it is possible to avoid suppression of expression of a target protein. Therefore, it is possible to detect expression of a desired protein more clearly.

A procedure for constructing a desired vector by causing a gene encoding a target protein and the second fluorescent protein to be operably linked to the second promoter is well known in the art. Further, a method for introducing an expression vector into a host is also well known in the art.

Therefore, reading the specification, a person skilled in the art could construct an expression vector appropriately, observe fluorescence from the fluorescent protein constituting the fusion protein in a seed, and observe fluorescence from the second fluorescent protein in a target tissue. For example, by constructing a vector such as pFAST-R05 and pFAST-R06 which are modified destination vectors constructed in a later-mentioned Example, it is possible to make observation as above.

The DNA construct of the present invention is useful both as a selection marker and a codominant marker. Further, a selection marker kit including the DNA construct of the present invention is also encompassed in the scope of the present invention. The wording "kit" used herein indicates a single member in which a plurality of components are packaged. That is, the selection marker kit of the present invention may have reagents other than the DNA construct of the present invention. A person skilled in the art could easily understand what reagents would be used when using the DNA construct of the present invention as a selection marker.

### [2] Transgenic plant

The present invention also provides a transgenic plant to which the DNA construct is introduced. The transgenic plant of the present invention includes a gene which is operably linked to a seed-specific promoter and which encodes the fusion protein of the seed protein and the fluorescent protein.

The wording "transformant" used herein indicates not only cells, tissues, and organs, but also an organism itself. The transformant of the present invention may be any transformant as long as at least a gene encoding polypeptide constituting the fusion protein of the present invention is introduced and the fusion protein is expressed. That is, a transformant produced by means other than an expression vector is also encompassed in the technical scope of the present invention.

The wording "a gene is introduced" used herein indicates that a gene is introduced into a target cell (host cell) by a well known genetic engineering process (gene manipulation technique) in such a manner that the gene can be expressed in the target cell (i.e. transformant). In a case where the present invention is applied to an industrial field using plants, the present invention is applicable to various products (plants and crops produced in agriculture, forestry and marine products industry). Specific examples of such products and crops include grains (e.g. rice plant, wheat, and corn), timbers (e.g. pine, cedar, and cypress), vegetables, flowers and ornamental plants.

A plant to be transformed in the present invention indicates any of a whole plant body, a plant organ (e.g. leaf, petal, shaft, root, and seed), a plant tissue (e.g. epidermis, phloem, parenchyma, xylem, vascular bundle, palisade tissue, and spongy tissue), a plant culture cell, a plant cell in various forms (e.g. suspension culture cell), protoplast, a segment of a leaf, and a callus. The plant to be transformed is not particularly limited and may belong to either monocotyledonous class or dicotyledonous class. In one embodiment, the transgenic plant of the present invention may be at least one of a grown plant, a plant cell, a plant tissue, a callus, and a seed.

A gene is introduced into a plant by a transformation process well known by a person skilled in the art (e.g. Agrobacterium transformation). In a case of Agrobacterium transformation, a constructed expression vector for a plant is introduced into an appropriate Agrobacterium and aseptic culture lamina is infected with this strain by a method well known in the art (e.g. leaf disk method).

When the DNA construct of the present invention is introduced via a callus with use of the vector, it is possible to distinguish a homozygous seed from heterozygous seed in a seed group of the transformant with fluorescence as an indicator, allowing easily obtaining a homozygous seed. Agrobacterium containing the DNA construct of the present invention allows introducing the DNA construct into an infected plant only by a floral-dip process or a vacuum-infiltration process (by applying Agrobacterium to flower bud or shoot apical meristem), and therefore it is only required to collect seeds from the infected plant. As described above, with the present invention, it is possible to obtain a target seed with a very simple method without introduction via a callus. Introduction via a callus requires a complicated process such as a sterilizing process and is defective because of high probability of appearance of culture variants. However, use of the above method allows avoiding such a defect. Further, the present invention is advantageous in that the present invention can transform any tissue or organ by selecting an appropriate second promoter.

Whether a gene has been introduced into a plant or not may be confirmed by PCR, Southern hybridization, Northern hybridization etc.

Once a transgenic plant in which polynucleotide of the present invention is introduced into genome is obtained, it is possible to obtain offspring of the plant by gamogenesis or agamogenesis. Further, it is possible to obtain seeds, fruits, cutting, tuber, tuberous root, strain, callus, protoplast etc. from the plant, offspring thereof, or clones thereof, and to produce a large amount of the plant based on the seeds, the fruits, the cutting, the tuber, the tuberous root, the strain, the callus, the protoplast etc. thus obtained. Therefore, the present invention encompasses a plant to which the fusion protein is introduced in an expressible manner, offspring of the plant which has the same properties as the plant, or tissue derived from the plant or the offspring.

A method of the present invention for preparing a transgenic plant includes the steps of: transforming plants with use of a gene which is operably linked to a seed-specific promoter and which encodes a fusion protein of a seed protein and a fluorescent protein; and selecting, from the transformed plants, a plant in which the fusion protein is expressed.

In one embodiment, the method of the present invention for preparing a transgenic plant may include the following steps:
(1) preparing a DNA construct including a gene which is operably linked to a seed-specific promoter and which encodes a fusion protein of a seed protein and a fluorescent protein;
(2) introducing plant expression vectors to a plurality of Agrobacterium, respectively, the gene extracted from the DNA construct prepared in the step (1) being inserted to the plant expression vectors;
(3) applying the plurality of Agrobacterium prepared in the step (2) to flower buds (by floral-dip) so as to infect plants;
(4) collecting T1 seeds from the plants prepared in the step (3) which are infected with the plurality of Agrobacterium;
(5) detecting whether the T1 seeds collected in the step (4) emit fluorescence derived from the fluorescent protein or not and selecting plants with fluorescence as transgenic plants;
(6) culturing the transgenic plants selected in the step (5) and collecting T2 seeds so as to construct a seed library; and
(7) detecting whether the T2 seeds collected in the step (6) emit fluorescence derived from the fluorescent protein or not and selecting seeds with fluorescence. Note that the steps (5) and (7) may be a step of detecting whether extracts from individual T1 seeds (or T2 seeds) or extracts from plants obtained by growing individual T1 seeds (or T2 seeds) include a gene encoding the fusion protein or a gene encoding the fluorescent protein. As described above, the method of the present embodiment for preparing a transgenic plant may include the step of applying Agrobacterium including the DNA construct to a flower bud or a shoot apical meristem.

### [3] Method for selecting transgenic plant

Use of the DNA construct of the present invention allows easily selecting a seed in which a target protein is expressed. That is, the present invention also provides a method for selecting a transgenic plant. A method of the present invention for selecting a transgenic plant include the step of detecting that a gene encoding a fusion protein of a seed protein and a fluorescent protein exists in a seed, the gene being operably linked to a seed-specific promoter. In one aspect, the method of the present invention for selecting a transgenic plant may be considered as a step included in a method for preparing a transgenic plant, and may be the steps (5)-(7) included in the aforementioned method for preparing a transgenic plant. That is, in the method for selecting a transgenic plant, the step of detecting may include detecting fluorescence of the fluorescent protein from a seed or may include detecting a gene encoding the fusion protein or a gene encoding the fluorescent protein from a seed extract.

The method of the present invention for selecting a transgenic plant may further include the step of detecting that a gene which is operably linked to a seed-specific promoter and which encodes a second fluorescent protein exists in a seed. This step may be carried out in such a manner that a second gene encoding a target protein and a gene encoding the second fluorescent protein are operably linked to the seed-specific promoter so as to construct a desired vector and then the vector is introduced into a host and seeds derived from the host are observed with a fluorescent microscope etc.

The method of the present invention for selecting a transgenic plant may further include the step of detecting that a gene which is operably linked to a second promoter and which encodes a second fluorescent protein exists in a target tissue.

This step may be carried out in such a manner that the second gene and a gene encoding the second fluorescent protein are operably linked to the second promoter so as to construct a desired vector and then the vector is introduced into a host and tissues in which a target protein is to be expressed are observed with a fluorescent microscope etc.

### [4] Method for producing protein

The present invention further provides a method for producing a protein. A method of the present invention for producing a protein is a method for producing a protein in a transgenic plant, including the steps of: (a) inserting, to a DNA construct including a first gene which is operably linked to a seed-specific promoter and which encodes a fusion protein of a seed protein and a fluorescent protein, a second gene encoding a target protein; and (b) introducing the DNA construct obtained in the step (a) to a plant. In the method, the second gene may be operably linked to the seed-specific promoter or may be operably linked to a second promoter for expressing in a target tissue a protein encoded by the second gene.

It is preferable to arrange the method of the present invention for producing a protein so as to further include the step of purifying a protein from an extract liquid of a transgenic plant (e.g. cells or tissues). The step of purifying a protein is preferably a step of preparing a cell extract liquid from cells or tissues by a well known method (e.g. a method of destroying cells or tissues and thereafter centrifuging the cells or the tissues so as to collect a soluble fraction) and thereafter purifying a protein from the cell extract liquid by a well known method (e.g. affinity purification using an antibody to a target protein, ammonium sulfate precipitation or ethanol precipitation, acid extraction, anion-exchange chromatography or cation-exchange chromatography, phosphocellulose chromatography, hydrophobic interaction chromatography, affinity chromatography, hydroxyapatite chromatography, and lectin chromatography), but the step of purifying a protein is not limited to this. it is most preferable to carry out high performance liquid chromatography (HPLC) for purification.

The method of the present invention for producing a protein is use of the transformant. Accordingly, a method for producing a protein including the step of the method for preparing the transformant shown in Embodiments is also encompassed in the technical scope of the present invention. In one embodiment, in the method of the present invention for producing a protein, floral-dip or vacuum-infiltration is carried out when introducing a target gene into a plant.

### [Examples]

### [1] Material and method

A reagent used in Examples was purchased from NACALAI TESQUE, INC. or Wako Pure Chemical Industries, Ltd. unless otherwise stated.

### [1-1] Plant material and growth condition

A plant material used in Examples was an ecotype Col-0 of Arabidopsis thaliana. A culture medium used here was a solid culture medium in which Murashige and Skoog Plant Salt Mixture was mixed with agarose and adjusted (MS culture medium). Agarose was controlled so that a final concentration thereof was 0.9% (w/w). Further, sucrose was added appropriately so that a final concentration thereof was 0-1%. In order to sterilize the surface of seeds, the seeds were subjected to a 70% ethanol treatment for 10 min, and then washed with 99% ethanol once. The seeds were sown on the culture medium aseptically, and were appropriately subjected to a low-temperature water absorption process at a dark place at 4°C for 3 days. Thereafter, the seeds were cultured at 22°C under a consecutive bright condition. A phytotron (SANYO growth chamber MLR-350) and a white fluorescent lamp (FL40SS·W/37, 40 type, 37 watt) were used for the culture.

The plant cultured on the solid culture medium was transferred to a plant pot (Yamato plastic Co., Ltd., KANEYA CO., LTD.) in which vermiculite (GL size, NITTAI Co., Ltd.) was put, and then the plant was cultured at 22°C under a long-day condition (light period: 16 hours, dark period: 8 hours). Water was given approximately once a week, and at the same time a solution derived from a stock solution of HYPONeX (HYPONeX JAPAN CORP., LTD.) by approximately one thousand dilution was given.

### [1-2] Preparation of antibody specific to CLO3

A portion of CLO3 which portion had a specific amino acid sequence having little homology with other protein was chemically synthesized with use of Peptide Synthesizer model 431 A (Applied Biosystems).

The synthesized peptide sequence is as follows.
CLO3: CVTSQRKVRNDLEETL (SEQ ID NO: 11)

The synthesized peptide was crosslinked with BSA with use of 3-maleimidobenzoic acid N-hydroxysuccinimide ester (Sigma-Aldrich). The peptide crosslinked with BSA was hypodermically injected, along with complete Freund's adjuvant which served as an adjuvant, into rabbits. The peptide crosslinked with BSA was additionally injected, along with incomplete Freund's adjuvant, into the rabbits four times, every two weeks after three weeks had elapsed from start of immune. An antibody was purified from a blood gathered from a rabbit one week after the last additional injection.

### [1-3] SDS-PAGE and CBB staining

SDS-PAGE was carried out in accordance with the method disclosed in Laemmli et al. J. Mol. Biol. 47, 69-85 (1970). A protein sample was suspended in a SDS sample buffer (4 weight % SDS, 100 mM Tris-HCl, 10 weight % 2-mercaptoethanol, 20 weight % glycerol, 0.1 % BPB (individual numerals indicate final concentrations of respective components in the sample solution), and heated at 95°C for 5 min. Thereafter, the heated protein sample was applied to 7.5-15% acrylamide gradient gel (BIO CRAFT). The gel was subjected to electrophoresis and then stained with a CBB stain solution (0.25 weight % of Coomassie blue R250, 45% methanol, 10% acetic acid) for one hour. Thereafter, the gel was destained with a destain solution A (45% methanol, 10% acetic acid) for one hour and with a destain solution B (5% methanol, 7% acetic acid) for twelve hours, so that a band of the protein was detected.

### [1-4] Immunoblotting

SDS-PAGE was carried out in the same manner as above with use of 15% acryl amide gel. After the gel was subjected to electrophoresis, the gel was immersed in a transfer solution (100 mM Tris-glycine (pH6.8), 20% methanol) and shaken for 5 min, and then provided between a nylon membrane having been subjected a pre-process using the same solution and a filter paper. A protein in the gel was electrically transferred onto the nylon membrane (Immunobilin-P, MILLIPORE) with use of a semi-dry blotter (Bio Craft) under a condition of 2mA/cm².

The nylon membrane to which the protein had been transferred was shaken in a TBS-T containing 5 weight % skim milk (50 mM Tris-HCl (pH7.5), 150 mM NaCl, 0.05 weight % Tween 20) for 30 min so that a blocking process was carried out. The nylon membrane having been subjected to blocking was shaken in a TBS-T containing an appropriately diluted antibody (dilution rate: 1/2000 for OLE1 antibody, 1/5000 for OLE2 antibody, 1/5000 for CLO3 antibody) for 1 hour. Subsequently, the membrane was washed with TBS-T three times for 5 min. Thereafter, the membrane was shaken in a TBS-T containing Goat Anti-Rabbit IgG-horseradish peroxidase (HRP) conjugate (ImmunoPure Goat Anti-Rabbit IgG [F(ab')2], Peroxidase Conjugated, PIERCE) for 30 min. Thereafter, the nylon membrane was washed once for 15 min and washed 3 times for 5 min, and then colored with an ECL kit (GE Healthcare) so that a band of the protein was detected with LAS-3000 (FUJI FILM)) .

### [2] Production of CLO3-excessively expressing transgenic plant having OLE1GFP marker

A transgenic plant excessively expressing CLO3 was produced under the control of Cauliflower mosaic virus 35S promoter (hereinafter abbreviated as 35S promoter). As a transformation selection marker for a plant, a fusion gene marker of OLE1 and GFP (OLE1GFP marker) was used. A construct was prepared by the method of Gateway Technology (Invitrogen).

### [2-1] Preparation of modified destination vector pBGWF7

A destination vector pBGWF7 (Pant System Biology) has a code region for GFP-GUS fusion protein at a downstream of a Gateway multicloning site. pBGWFS7 was treated with a restriction enzyme Nru1, so that a modified destination vector pBGWF7 in which a GUS code region in the vector was removed was prepared.

### [2-2] Cloning of OLE1 gene

In order to express a protein in which GFP is fused with a C-terminus of OLE1 (OLE1GFP), approximately 2kb of upstream of a code region of a protein in the OLE1 gene was used as a promoter region. In order to fuse GFP with a C-terminus of an OLE1 protein, stop codon of an OLE1 code region was removed. In order to prevent frame shift, one base of guanine was added to reverse primer. An OLE1 gene was amplified from a template of Col-0 genome with use of TOYOBO KOD-plus-Polymerase, subcloned into pENTER/D-TOPO (Invitrogen), and an entry vector pOLE1 was prepared. A base sequence of the entry vector pOLE1 was confirmed by ABI BigDye Terminator v3.1 Cycle Sequencing Kit.

The primers used here are as follows.
OLE1_Fwd, 5'-CACCCTACTTAGATCAACACATAAA-3' (SEQ ID NO: 12)
OLE1_Rev, 5'-GAGTAGTGTGCTGGCCACCACG-3' (SEQ ID NO: 13)

### [2-3] Preparation of OLE1GFP construct

According to the method of Gateway Technology, an LR reaction was carried out between the modified destination vector pBGWD7 and the entry vector pOLE1 so that an expression vector pB-OLE1GFP construct was prepared.

### [2-4] Preparation of modified destination vector pB-OLE1GFP-2GW7

The destination vector pH2GW7 (Plant System Biology) was treated with restriction enzyme Aat2 and thus 3kDa of DNA fragments containing 35S promoter, gateway multicloning site, and 35S terminator were obtained. The expression vector pB-OLE1GFP was also treated with Aat2, and further treated with alkaline phosphatase to prevent intramolecular binding, and thus DNA fragments were obtained. the two fragments were ligated with each other to prepare the modified destination vector pB-OLE1GFP-2GW7 (Fig. 1, upper figure).

### [2-5] Cloning of CLO3 gene

As a gene to be introduced into the modified destination vector pB-OLE1GFP-2GW7, CLO3 which is an isoform of caleosin which is an oil body protein, was used (Chen et al. Plant Cell Physiol. 40, 1079-1086 (1999), Naested et al. Plant Mol. Biol. 44, 463-476 (2000), Frandsen et al. Physiol. Plant 112, 301-307 (2001), Hanano et al. J. Biol. Chem. 281, 33140-33151 (2006)). CLO3 mRNA was induced in a vegetative organ as a result of dry stress, salt stress, and abscisic acid treatment (Takahashi et al. Plant Cell Physiol. 41, 898-903 (2000)). Observation of accumulation of CLO3 protein showed that seedling on seventh day did not have any accumulation (Fig. 4(a)).

A region ranging from a start codon to a stop codon of a CLO3 gene was amplified from a Col-0 genome as a template with use of TOYOBO KOD-plus-Polymerase, and subcloned into pENTER/D-TOPO (Invitrogen), and thus an entry vector pCLO3 was prepared. A base sequence of the entry vector pCLO3 was confirmed with use of ABI BigDye Terminator v3.1 Cycle Sequencing Kit.

The primers used here are as follows.
CLO3_Fwd; 5'-CACCATGGCAGGAGAGGCAGAGGCTT-3' (SEQ ID NO: 14)
CLO3_Rev;5'-TTAGTCTTGTTTGCGAGAATTGGCCC-3' (SEQ ID NO: 15)

### [2-6] Preparation of pB-OLE1GFP-35S-CLO3 construct having OLE1GFP marker

An LR reaction was carried out between an entry vector pCLO3 and pB-OLE1GFP-2GW7 according to the method of Gateway Technology, so as to prepare an expression vector pB-OLE1GFP-35S-CLO3 construct (Fig. 1, lower figure). pB-OLE1GFP-2GW7 has a cloning site at downstream of a 35S promoter, and is capable of excessively expressing a target gene under the control of the 35S promoter as a result of the LR reaction.

### [2-7] Further construction of modified destination vector

As a modified destination vector including the prepared OLE1GFP fusion gene, a general vector (pH-OLE1GFP-GW), a vector for RNAi (pH-OLE1GFP-7GW1WG2(I)), and a vector for promoter analysis (pK-OLE1GFP-GWFS7) were further prepared in addition to the vector for excessively expressing a target gene under the control of 35S promoter (pB-OLE1GFP-2GW7) (Fig. 7).

3.5kb area containing the OLE1GFP fusion gene and the terminator 35S was amplified by PCR with use of TOYOBO KOD-plus-Polymerase from pB-OLE1GFP-2GW7 as a template. At that time, a recognition sequence of a restriction enzyme Apa1 or Spe1 was added to a primer, so that the recognition sequence of Apa1 or Spe1 was added at upstream and downstream of DNA fragments containing the OLE1GFP fusion gene and the terminator 35S. Each of the obtained fragments was subcloned into pENTER/D-TOPO (Invitrogen) so as to prepare entry vectors pOLE1GFP-Apa1 and pOLE1GFP-Spe1. Base sequences of the entry vectors pOLE1GFP-Apa1 and pOLE1GFP-Spe1 were confirmed with use of ABI BigDye Terminator v3.1 Cycle Sequencing Kit. The primers used here are as follows.
OLE1GFP-Apa1_Fwd; 5'-CACCGGGCCCTACTTAGATCAACACATAAA-3' (SEQ ID NO: 16)
OLE1GFP-Apa1_Rev;5'-GGGCCCTCGCATGCCTGCAGGTCACTGGAT-3' (SEQ ID NO: 17)
OLE1GFP-Spe1_Fwd;5'-CACCACTAGTTAGTAAGTGAAGAACCACAA-3' (SEQ ID NO: 18)
OLE1GFP-Spe1_Rev;5'-ACTAGTCGCATGCCTGCAGGTCACTGGAT-3' (SEQ ID NO: 19)

A destination vector pHGW (Plant System Biology) was digested with the restriction enzyme Apa1, and the resulting DNA fragments were treated with alkaline phosphatase in order to prevent intermolecular binding. Similarly, the entry vector pOLE1GFP-Apa1 was digested with the restriction enzyme Apa1 so as to purify 3.5kb of DNA fragments containing the OLE1GFP fusion gene and the terminator 35S. These two fragments were ligated with each other so as to prepare a modified destination vector pH-OLE1GFP-GW as a general vector.

A destination vector pH7GWIWG2(I) (Plant System Biology) was digested with the restriction enzyme Apa1, and the resulting DNA fragments were treated with alkaline phosphatase in order to prevent intermolecular binding. Similarly, the entry vector pOLE1GFP-Apa1 was digested with the restriction enzyme Apa1 so as to purify 3.5kb of DNA fragments containing the OLE1GFP fusion gene and the terminator 35S. These two fragments were ligated with each other so as to prepare a modified destination vector pH-OLE1GFP-7GWIWG2(I) as a vector for RNAi.

A destination vector pKGWFS7 (Plant System Biology) was digested with the restriction enzyme Spe1, and the resulting DNA fragments were treated with alkaline phosphatase in order to prevent intermolecular binding. Similarly, the entry vector pOLE1GFP-Spe 1 was digested with the restriction enzyme Spe1 so as to purify 3.5kb of DNA fragments containing the OLE1GFP fusion gene and the terminator 35S. These two fragments were ligated with each other so as to prepare a modified destination vector pK-OLE1GFP-GWFS7 as a vector for promoter analysis.

### [2-8] Production of Arabidopsis thaliana expressing pB-OLE1GFP-35S-CLO3

The prepared expression vector pB-OLE1GFP-35S-CLO3 was introduced into Agrobacterium (Agrobacterium tumefaciens GV3101 strain) by electroporation and wild type Co1-0 was transformed by floral-dip (Daimon et al. Third revised edition, Experiment protocol for model plants, Shujunsha, 149-154 [0095]). A transformant was selected with the OLE1GFP marker as an indicator. This transgenic plant is referred to as 35S: CLO3 (OLE1GFP).

Further, a series including introduced genes at 1 locus was isolated so as to obtain a series including introduced genes homozygously. With respect to each homozygous seed, expression of a protein was confirmed by immunoblotting.

### [2-9] Production of transgenic plant expressing OLE1GFP

The aforementioned expression vector pB-OLE1GFP construct was introduced into Agrobacterium (Agrobacterium tumefaciens GV3101 strain) by electroporation, and wild type Col-0 was transformed by floral-dip. A transformant was selected with an OLE1GFP marker as an indicator. A series including introduced genes at 1 locus was isolated so as to obtain a series including introduced genes homozygously. With respect to each homozygous seed, expression of a protein was confirmed by immunoblotting. [2-10] Observation and selection of OLE1GFP expressing seeds

A seed group was observed with a fluorescent microscope and existence of seeds having GFP fluorescence and a segregation ratio thereof were confirmed. When selecting a seed having GFP fluorescence, the seed was selected from the group with use of a pick with a little moist end. When measuring fluorescence intensity of the seed, the seed was photographed and fluorescence intensity of the seed was measured based on the image with use of Photoshop Elements 5.0. The seed was sown on an MS culture medium. Further, according to necessity, the seed was sown on a culture medium containing Glufosinate-ammonium (10mg/L).

### [3] Production of modified destination vector pFAST-R vector

As a transformation selection marker for a plant, there was prepared a modified destination vector having a fusion gene marker (OLE1TagRFP marker) of OLE1 and Tag RFP (Evrogen JSC, Moscow, Russia) (Merzlyak et al., Bright monomeric red fluorescent protein with an extended fluorescence lifetime, Nat. Methods, vol.4, 555-7, 2007). The OLE1TagRFP marker consists of an OLE1 promoter, OLE1-TagRFP fusion gene, and an NOS terminator.

### [3-1] Cloning of OLE1 gene, TagRFP, and NOS terminator

In order to express a protein in which TagRFP is fused with a C-terminus of OLE1 (OLE1TagRFP), approximately 2kb of upstream of a coding region for a protein of an OLE1 gene was used as a promoter region. In order to fuse TagRFP with a C-terminus of an OLE1 protein, a stop-codon of an OLE1 coding region was removed. Approximately 2.2kb of the OLE1 gene was amplified from pB-OLE1GFP as a template with use of TOYOBO KOD-plus-Polymerase. Further, approximately 0.7kb of TagRFP fragments and approximately 0.2kb of NOS terminator fragments were amplified.

The primers used here are as follows.
OLE1_Fwd2,5 '-CACCACTAGTGTATGTAGGTATAGTAACAT-3' (SEQ ID NO: 20)
OLE1_Rev2,5'-CAGCTCGCTCATAGTAGTGTGCTGGCCACC-3' (SEQ ID NO: 21)
TagRFP_Fwd,5'-CAGCACACTACTATGAGCGAGCTGATTAAG-3' (SEQ ID NO: 22)
TagRFP_Rev,5'-TGTTTGAACGATTCACTTGTGCCCCAGTTT-3' (SEQ ID NO: 23)
NOST_Fwd,5'-GGGCACAAGTGAATCGTTCAAACATTTGGC-3' (SEQ ID NO: 24)
NOST_Rev,5'-ACTAGTGATCTAGTAACATAGATGACACC-3' (SEQID NO: 25)

### [3-2] Preparation of OLE1TagRFP marker

Using fragments of the OLE1 gene, fragments of the TagRFP, and fragments of the NOS terminator which were amplified in [3-1], approximately 3.5kb of OLE1TagRFP marker fragments consisting of OLE1 promoter, OLE1-TagRFP fusion gene and NOS terminator were amplified with use of TOYOBO KOD-plus-Polymerase. At that time, a recognition sequence of a restriction enzyme Spe1, Hind3, or Apa1 was added to a primer and the recognition sequence of a restriction enzyme Spe1, Hind3, or Apa1 was added at upstream and downstream of the OLE1TagRFP marker fragments. Each of the obtained fragments was subcloned into pENTER/D-TOPO (Invitrogen) so as to prepare entry vectors pOLE1TagRFP-Spe1, pOLE1TagRFP-Hind3, and pOLE1TagRFP-Apa1. Base sequences of the entry vectors pOLE1TagRFP-Spe1, pOLE1TagRFP-Hind3, and pOLE1TagRFP-Apa1 were confirmed with use of ABI BigDye Terminator v3.1 Cycle Sequencing Kit.

The primers used here are as follows.
FAST-R_Spe 1 Fwd, 5'-CACCACTAGTGTATGTAGGTATAGTAACAT-3' (SEQ ID NO: 26)
FAST-R_Spe 1 Rev, 5'-ACTAGTGATCTAGTAACATAGATGACACC-3' (SEQ ID NO: 27)
FAST-R_Hind3Fwd,5'-CACCAAGCTTCAAGTGTATGTAGGTATAGT-3' (SEQ ID NO: 28)
FAST-R_Hind3Rev,5'-AAGCTTGATCTAGTAACATAGATGACACC-3' (SEQ ID NO: 29)
FAST-R_Apa1 Fwd,5'-CACCGGGCCCTTCAAGTGTATGTAGGTATA-3' (SEQ ID NO: 30)
FAST-R_Apa1Rev,5'-GGGCCCATCTAGTAACATAGATGACACC-3' (SEQ ID NO: 31)

### [3-3] Preparation of modified destination vector pHGWF7

A destination vector pHGWFS7 (Plant System Biology) has a coding region for a GFP-GUS fusion protein at downstream of a gateway multicloning site. pHGWFS7 was treated with a restriction enzyme Nru1 so as to prepare a modified destination vector pHGWF7 in which a GUS region in the vector was removed.

### [3-4] Preparation of modified destination vector pFAST-RO1

A destination vector pHGW was treated with a restriction enzyme Spe1 and the resulting DNA fragments were treated with alkaline phosphatase in order to prevent intermolecular binding. Similarly, an entry vector pOLE1TagRFP-Spe1 was treated with the restriction enzyme Spe1 so as to purify 3.5kDa of DNA fragments containing OLE1-TagRFP fusion gene and NOS terminator. These two fragments were ligated with each other so as to prepare a modified destination vector pFAST-RO1 which was a general vector (Fig. 8).

### [3-5] Preparation of modified destination vector pFAST-R02

A destination vector pBGWFS7 (Plant System Biology) was treated with a restriction enzyme Apa1 and the resulting DNA fragments were treated with alkaline phosphatase in order to prevent intermolecular binding. Similarly, an entry vector pOLE1TagRFP-Apa1 was treated with the restriction enzyme Apa1 so as to purify 3.5kDa of DNA fragments containing OLE1-TagRFP fusion gene and NOS terminator. These two fragments were ligated with each other so as to prepare a modified destination vector pFAST-R02 which was a vector for excessively expressing a target gene under the control of 35S promoter (Fig. 8).

### [3-6] Preparation of modified destination vector pFAST-R03

A destination vector pH7GWIWG2(I) was treated with a restriction enzyme Apa1 and the resulting DNA fragments were treated with alkaline phosphatase in order to prevent intermolecular binding. Similarly, an entry vector pOLE1TagRFP-Apa1 was treated with the restriction enzyme Apa1 so as to purify 3.5kDa of DNA fragments containing OLE1-TagRFP fusion gene and NOS terminator. These two fragments were ligated with each other so as to prepare a modified destination vector pFAST-R03 which was a vector for (knocking down) RNAi (Fig. 8).

### [3-7] Preparation of modified destination vector pFAST-R05

A destination vector pGWB405 (Nakagawa et al., Development of series of gateway binary vectors, pGWBs, for realizing efficient construction of fusion genes for plant transformation, J. Biosci. Bioeng., 2007, vol. 104, 34-41) was treated with a restriction enzyme Hind3 and the resulting DNA fragments were treated with alkaline phosphatase in order to prevent intermolecular binding. Similarly, an entry vector pOLE1TagRFP-Hind3 was treated with the restriction enzyme Hind3 so as to purify 3.5kDa of DNA fragments containing OLE1-TagRFP fusion gene and NOS terminator. These two fragments were ligated with each other so as to prepare a modified destination vector pFAST-R05 for expressing a target protein in which GFP was fused with a C-terminus (Fig. 8).

### [3-8] Preparation of modified destination vector pFAST-R06

A destination vector pGWB406 (Nakagawa et al., J. Biosci. Bioeng., 2007, vol. 104, 34-41) was treated with a restriction enzyme Hind3 and the resulting DNA fragments were treated with alkaline phosphatase in order to prevent intermolecular binding. Similarly, an entry vector pOLE1TagRFP-Hind3 was treated with the restriction enzyme Hind3 so as to purify 3.5kDa of DNA fragments containing OLE1-TagRFP fusion gene and NOS terminator. These two fragments were ligated with each other so as to prepare a modified destination vector pFAST-R06 for expressing a target protein in which GFP was fused with an N-terminus (Fig. 8).

### [3-9] Preparation of modified destination vector pFAST-R07

A destination vector pHGWF7 was treated with a restriction enzyme Spe1 and the resulting DNA fragments were treated with alkaline phosphatase in order to prevent intermolecular binding. Similarly, an entry vector pOLE1TagRFP-Spe1 was treated with the restriction enzyme Spe1 so as to purify 3.5kDa of DNA fragments containing OLE1-TagRFP fusion gene and NOS terminator. These two fragments were ligated with each other so as to prepare a modified destination vector pFAST-R07 for expressing a target protein in which GFP was fused with a C-terminus (Fig. 8).

An LR reaction was carried out between an entry vector pCLO3 and pFAST-R02 according to the method of Gateway Technology, so as to prepare an expression vector pB-35S-CLO3-OLE1TagRFP construct. pFAST-R02 has a cloning site at downstream of a 35S promoter, and is capable of excessively expressing a target gene under the control of the 35S promoter as a result of the LR reaction.

### [3-11] Production of Arabidopsis thaliana expressing pB-35S-CLO3-OLE1TagRFP

The prepared expression vector pB-35S-CLO3-OLE1TagRFP was introduced into Agrobacterium (Agrobacterium tumefaciens GV3101 strain) by electroporation and wild type Co1-0 was transformed by floral-dip (Daimon et al. Third revised edition, Experiment protocol for model plants, Shujunsha, 149-154 [0111]). A transformant was selected with the OLE1TagRFP marker as an indicator. The result is shown in Fig. 9.

### [3-12] Preparation of pB-OLE1TagRFP-35S-GFPCLO3 construct having OLE1TagRFP marker

An LR reaction was carried out between an entry vector pCLO3 and pFAST-R06 according to the method of Gateway Technology, so as to prepare an expression vector pB-OLE1TagRFP-35S-GFPCLO3 construct. PFAST-R06 has a GFP gene and a cloning site at downstream of a 35S promoter, and is capable of excessively expressing a fusion protein derived from the GFP gene and the target gene under the control of the 35S promoter as a result of the LR reaction.

### [3-13] Production of Arabidopsis thaliana expressing pB-OLE1TagRFP-35S-GFPCLO3

The prepared expression vector pB-OLE1TagRFP-35S-GFPCLO3 was introduced into Agrobacterium (Agrobacterium tumefaciens GV3101 strain) by electroporation and wild type Co1-0 was transformed by floral-dip (Daimon et al. Third revised edition, Experiment protocol for model plants, Shujunsha, 149-154 [0114]). A transformant was selected with the OLE1TagRFP marker as an indicator. This transgenic plant is referred to as 35S:: GFP-CLO3 (FAST-R06). a series including introduced genes at 1 locus was isolated so that a series including introduced genes homozygously was obtained.

A leaf of 35S:: GFP-CLO3 (FAST-R06) was observed with a confocal laser microscope (LSM510 META; Carl Zeiss, Jena, Germany) and GFP fluorescence in a cell was photographed. Laser used here was 488-nm line of a 40-mV Ar/Kr laser. A differential interference contrast (DIC) image was photographed at the same time. The results are shown in Figs. 10 and 11.

### [4] Result and analysis

The DNA construct of the present invention is shown in Fig. 1. The upper figure in Fig. 1 shows a vector (pB-OLEGFP-2GW7) for preparing a plant excessively expressing a target gene under the control of a CaMV35S promoter, and the lower figure in Fig. 1 shows a vector (pB-OLE1GFP-35S:: CLO3) for excessively expressing CLO3 as one embodiment. In the figures, LB indicates Left Boarder, RB indicates Right Boarder, Bar indicates a Basta gene, p35s indicates a CaMV35S promoter, t35s indicates a CaMV35S terminator, CmR indicates a chloramphenicol-resistance gene, and ccdB indicates Escherichia Coli gyrase inhibiting protein.

Fig. 2 shows the result of observing, with a fluorescent microscope, seeds of a plant series to which a vector for excessively expressing CLO3 (pB-OLE1GFP-35S:: CLO3) was introduced. Initially, a wild type Co1-0 (T0 plant) was transformed with use of pB-OLE1GFP-35S:: CLO3 into a plant (35SCLO3 (OLE1GFP)). Seed groups T1 and T2 and a homozygous seed group T3 each obtained from the plant were observed with a fluorescent microscope. (a) indicates GFP fluorescence, and (b) indicates a bright-field image. In the seed group T1, some seeds having GFP fluorescence were observed (indicated by pike in figure). Selected T1 seeds of 35S: CLO3 (OLE1GFP) were cultured and the resulting T2 seed group was observed with a fluorescent microscope. The result of the observation showed that seeds with GFP fluorescence (GFP+) and seeds without GFP fluorescence (GFP-) coexisted (Fig. 2, T2 seeds). Further, in the next-generation T3 homozygous seed group obtained by culturing T2 seeds, all seeds had GFP fluorescence (Fig. 2, T3 seeds).

Fig. 3 shows segregation ratios of the T2 seed group and the T3 homozygous seed group T3 of the 35SCLO3 (OLE1GFP) plant. With respect to the T2 seed group and the T3 homozygous seed group T3 of 35SCLO3 (OLE1GFP), the number of seeds with GFP fluorescence (GFP+) and the number of seeds without GFP fluorescence (GFP-) were counted. As shown in Fig. 3, in #1 series, #5 series, and #6 series of the T2 seed group, the segregation ratio of GFP+ to GFP- was approximately 3 to 1. Further, in #2 series of the T2 seed group, the segregation ratio of GFP+ to GFP- was 15 to 1. From these segregation ratios, it was inferred that the series with the segregation ratio of 3 to 1 had pB-OLE1GFP-35S:: CLO3 construct inserted into 1 locus and the series with the segregation ratio of 15 to 1 had pB-OLE1GFP-35S:: CLO3 construct inserted into 2 locus.

In order to examine whether the OLE1GFP gene has a selective ability similar to that of a drug selection marker, the number of seeds with resistance to a drug (Glufosinate-ammonium) (barR) was counted and it was confirmed whether existence/absence of GFP fluorescence correspond to existence/absence of drug-resistance. In four series of the T2 seed group (up to 300 seeds) of 35S: CLO3 (OLE1GFP), all seeds with GFP fluorescence (GFP+) had drug-resistance (barR), and seeds without GFP fluorescence (GFP-) did not have drug-resistance (Fig. 3). In the T3 homozygous seed group, all seeds had GFP fluorescence, and exhibited drug-resistance (Fig. 3). This shows that the OLE1GFP fusion gene is not only usable as a visual selection marker but also has a selective ability similar to that of a drug-selection marker. The OLE1GFP fusion gene is hereinafter referred to as an OLE1GFP marker.

Accumulation of the CLO3 protein in the obtained transformant was examined. Fig. 4 shows the result of confirming expression of CLO3 in seeds with observed GFP fluorescence in the seed group of the 35SCLO3(OLE1GFP plant). (a) indicates the result of culturing seeds with observed GFP fluorescence, seeds without observed GFP fluorescence, and T2 homozygonous series in each of wild type plant Co1-0, OLE1GFP plant, and T2 series of 35SCLO3(OLE1GFP) plant and examining expression of CLO3 in seedling on seventh day by immunoblotting. (b) indicates the number of seeds whose expression of CLO3 was observed in seedling on seventh day as a result of culturing 16 seeds with observed GFP fluorescence and 17 seeds without observed GFP fluorescence in T2 series of 35SCLO3(OLE1GFP) plant. In the wild type plant Col-0, expression of CLO3 was not observed in seedling on seventh day. Further, in the 35SCLO3(OLE1GFP) plant, expression of CLO3 was not observed in seedling on seventh day of a plant cultured from seeds without observed GFP fluorescence. On the other hand, in a plant cultured from seeds with observed GFP fluorescence, expression of CLO3 was not observed in seedling on seventh day. These results indicate that expression of CLO3 is induced by 35S promoter and use of OLE1GFP marker allows correctly selecting transformants.

Fig. 5 is a drawing showing transition of fluorescence in germinated OLE1GFP. GFP fluorescence in OLE1GFP plant, seeds and seedlings of T3 homozygous series in 35SCLO3(OLE1GFP) plant were observed with a fluorescent microscope. Observation was made on 0^{th} day, 3^{rd} day, and 5^{th} day after changing the temperature to 22°C. Seedlings on 3^{rd} day after changing the temperature to 22°C exhibited reduced GFP fluorescence compared with that on 0^{th} day after the change. Seedlings on 5^{th} day after the change exhibited no GFP fluorescence. As described above, dry seeds exhibited the strongest OLE1GFP fluorescence, which was gradually reduced after germination, and substantially disappeared on 5^{th} day. This seems to be because expression of OLE1 is limited to a seed ripening period (Kim et al. J. Biol. Chem. 277, 22677-22684 (2002)). Observation of intracellular fluorescence was made with a confocal laser scan microscope, and no fluorescence was observed in roots, leaves, and shafts (the result is not shown). In contrast thereto, in unripen seed cells and dry seed cells, GFP fluorescence was observed in areas other than PSV (the result is not shown). It is considered that this was because OLE1GFP fluorescence existed on an oil body. The above results show that OLE1GFP fusion gene is very useful as a visual selection marker for selecting a target gene.

Among seeds with GFP fluorescence in the T2 seed group (insertion was made at 1 locus) of 35S: CLO3(OLE1GFP), there existed seeds with high fluorescence intensity and seeds with low fluorescence intensity (Fig. 2). It is inferred from this fact that seeds with high fluorescence intensity belong to a homozygous series and seeds with low fluorescence intensity belong to a heterozygous series. In order to confirm this, GFP fluorescence intensity of individual T2 seeds was measured. Further, individual seeds were cultured and a segregation ratio of next-generation was observed, so as to examine mating types of individual seeds whose GFP fluorescence was measured (approximately 150 seeds per line). Fig. 6 shows a relation between GFP fluorescence intensity of T2 seeds of the 35SCLO3(OLE1GFP) plant and the genetic type of a transformed gene. GFP fluorescence intensity of a seed of T2 seeds (#1 series) of the 35ScLO3(OLE1GFP) plant was measured and the genetic type of a transformed gene of the seed was examined. The genetic type was determined by culturing a plant and examining a segregation ratio of GFP fluorescence of the resulting seeds. With respect to each of a non-transformant, a heterozygous series, and a homozygous series, a histogram was made with its vertical axis indicating the number of seeds and its lateral axis indicating GFP fluorescence intensity. The histograms indicative of GFP fluorescence intensity made for the homozygous series, the heterozygous series, and the non-transformant, respectively, show that GFP fluorescence intensity is higher in the heterozygous series seed group than in the non-transformant seed group, and higher in the homozygous series seed group than in the heterozygous series seed group. Similar results were obtained for #5 series and #6 series (the results are not shown). This suggests that by selecting seeds with very high fluorescence intensity from the T2 seed group, it is possible to select homozygous series seeds.

In order to obtain homozygous series seeds efficiently, it was calculated what percent of the top of the group with high fluorescent intensity in the T2 seed group should be selected. It was expected from the result of the calculation that in #1, #5, and #6 series, almost all of approximately top 5-10% of seeds with high GFP fluorescence intensity belong to homozygous series (the result is not shown). In contrast thereto, in a case where a drug-selection marker is used, it is impossible to distinguish T2 homozygous series from T2 heterozygous series. Consequently, in a case where a transformed gene is positioned at 1 locus (T2 homozygous series: T2 heterozygous series: non-transformant = 1: 2: 1), the probability that a seed selected by a drug belongs to a homozygous series is 33.3%. When four seeds are selected, the probability that none of the four seeds belong to homozygous series is 19.8%, showing that it is highly possible that use of a drug-selection marker cannot select homozygous series seeds, compared with use of the OLE1GFP marker. These results show that the OLE1GFP marker is useful as a codominant marker capable of distinguishing homozygous series from heterozygous series.

Fig. 9 shows the result of observation with a fluorescent microscope of seeds of a plant series to which an expression vector pB-35S-CLO3-OLE1TagRFP was introduced. Initially, a transgenic plant was obtained by transforming a wild type Co1-0 (T0 plant) with use of pB-35S-CLO3-OLE1TagRFP. T1 seed group obtained from the plant was observed with the fluorescent microscope. (a) indicates fluorescence of TagRFP, and (b) indicates a bright-field image. As shown in Fig. 9, in the T1 seed group, several seeds had red fluorescence of TagRFP. This shows that similarly with the OLE1GFP fusion gene, the OLE1TagRFP fusion gene is useful as a visual selection marker, too.

Fig. 10 shows the result of observation with a fluorescent microscope of T3 homozygous series seed group obtained from 35S:: GFP-CLO3 (FAST-R06). (a) indicates fluorescence of TagRFP, and (b) indicates a bright-field image. In the T3 homozygous series seed group obtained by culturing T1 seeds of 35S:: GFP-CLO3 (FAST-R06) and culturing the resulting T2 seed group, all seeds exhibited fluorescence of TagRFP (Fig. 10(a)).

Fig. 11 shows the result of observing expression of CLO3 in a leaf of 35S:: GFP-CLO3(FAST-R06) with GFP fluorescence as an indicator. (a) is an image showing the result of observing the leaf with a differential interference microscope, and (b) is an image showing the result of observing the leaf with a confocal laser microscope and detecting GFP fluorescence. (c) is an image obtained by overlapping the images of (a) and (b).

As shown in Fig. 8, pFAST-R06 includes an OLE1TagRFP fusion gene. Therefore, in 35S:: GFP-CLO3(FAST-R06), it is possible to confirm expression of a selection marker in a seed based on red fluorescence of TagRFP. On the other hand, in pFAST-R06, a gene encoding GFP which is a second fluorescent protein and a gene encoding CLO3 which is a target protein are operably linked to 35S promoter which is a second promoter. Accordingly, as shown in Figs. 11(b) and (c), expression of CLO3 in a leaf of 35S:: GFP-CLO3(FAST-R06) can be detected by green fluorescence distinctly from expression of a selection marker in a seed.

As described above, the DNA construct of the present invention is a novel selection marker for selecting a transgenic plant, and an expressed protein is a fusion protein of a seed protein derived from a plant and a fluorescent protein which is innoxious to an organism. In view of the above, the DNA construct of the present invention is a safe selection marker which is innocuous to an organism and an environment.

The DNA construct of the present invention is a selection marker which is more easy to use and is more useful than a general drug-resistance marker. In a case where a drug-resistance marker is used, selection of a transgenic plant or examination of a segregation ratio requires preparing a selective culture medium having a drug with an appropriate concentration and sowing seeds thereon. In contrast thereto, in a case where the DNA construct of the present invention which is a visual selection marker is used, preparation of a culture medium with a special composition and sowing seeds thereon are not required. Further, if genetic transformation is not carried out successfully, the failure can be known by observing fluorescence, which makes it unnecessary to sow seeds on a selective culture medium. Consequently, use of the DNA construct of the present invention allows reducing a drug and a culture medium.

In a case where a drug-resistance marker is used, selection of a T1 plant requires sowing a T1 seed group consisting of a large number of T1 seeds in a selective culture medium, which is very time-consuming. In contrast thereto, in a case where the DNA construct of the present invention is used, it is possible to carry out selection based on visual observation of dry seeds. This only requires sowing seeds which are surely T1 transformants. This reduces the number of seeds to be sown and so very efficient. Further, in this case, seeds may be cultured in a normal MS culture medium and may be planted directly in earth. Therefore, when a transformant with a phenotype which appears also in a heterozygous series, such as RNAi and excess expresser, is used, comparison between the transformant and a control plant can be carried out at the stage of a T1 transformant, thereby allowing prompt analysis. Further, it is possible to select a transformant which is too weak to be cultured in a selective culture medium.

In a case where a drug-resistance marker is used, it is impossible to distinguish a homozygous series from a heterozygous series in a T2 seed group. In contrast thereto, the DNA construct of the present invention is usable as a codominant marker, and therefore selection of seeds with high fluorescence intensity allows isolating a homozygous series with high probability, which shortens a time required to isolate the homozygous series by one generation.

Use of the DNA construct of the present invention allows producing a transgenic plant by floral-dip or vacuum-infiltration with use of Agrobacterium. Further, a plant to which the DNA construct of the present invention is applied is not limited as long as the plant accumulates a seed protein (oil body-localized protein in particular) in seeds, and therefore the DNA construct of the present invention is applicable to various plants. For example, it is reported that radish (Raphanus sativus) is a plant to which floral-dip or vacuum-infiltration is applicable (Curtis, I.S. and Nam, H.G. Transgenic Res. 10, 363-371 (2001)). It is inferred from this report that a brassicaceous plant which have oil seeds and accumulates an oil body-localized protein (oleosin) is a plant to which floral-dip or vacuum-infiltration is applicable, which indicates that the DNA construct of the present invention is widely applicable to a brassicaceous plant.

The embodiments and concrete examples of implementation discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

### Industrial Applicability

Use of the present invention allows obtaining a target transformant in a relatively short time without requiring a complicated process. Accordingly, the present invention is effectively used in breeding.

## Claims

1. A DNA construct, comprising a gene encoding a fusion protein of OLE1 and a fluorescent protein, the gene being operably linked to an oleosin promoter consisting of a base sequence represented by SEQ ID NO: 6,
the OLE1 consisting of an amino acid sequence represented by SEQ ID NO:1.

2. The DNA construct as set forth in claim 1, wherein
a second gene encoding a target protein and a gene encoding a second fluorescent protein are operably linked to the oleosin promoter, and
the second fluorescent protein is a protein that emits fluorescence with a color different from a color of fluorescence of the fluorescent protein constituting the fusion protein of the OLE1 and the fluorescent protein.

3. The DNA construct as set forth in claim 1, further comprising a second promoter for expressing a target protein in a target tissue.

4. The DNA construct as set forth in claim 3, wherein
a second gene encoding a target protein and a gene encoding a second fluorescent protein are operably linked to
the second promoter, and
the second fluorescent protein is a protein that emits fluorescence with a color different from a color of fluorescence of the fluorescent protein constituting the fusion protein of the OLE1 and the fluorescent protein.

5. Use of a DNA construct as set forth in any one of claims 1-4 for a selection marker.

6. A transgenic plant, to which a gene encoding a fusion protein of OLE1 and a fluorescent protein is introduced, the gene being operably linked to an oleosin promoter consisting of a base sequence represented by SEQ ID NO: 6,
the OLE1 consisting of an amino acid sequence represented by SEQ ID NO:1.

7. A method for selecting a transgenic seed, comprising the steps of:
applying Agrobacterium including a DNA construct as set forth in claim 1 to a flower bud so as to transform a plant and prepare a transgenic plant; and
detecting that a gene encoding a fusion protein of OLE1 and a fluorescent protein exists in a seed, the gene being operably linked to an oleosin promoter consisting of a base sequence represented by SEQ ID NO: 6,
the step of detecting including detecting fluorescence of the fluorescent protein from a seed, and
the OLE1 consisting of an amino acid sequence represented by SEQ ID NO:1.

8. The method as set forth in claim 7, further comprising the step of detecting that a gene which is operably linked to the oleosin promoter and which encodes a second fluorescent protein exists in a seed, the second fluorescent protein being a protein that emits fluorescence with a color different from a color of fluorescence of the fluorescent protein constituting the fusion protein of the OLE1 and the fluorescent protein.

9. The method as set forth in claim 7, further comprising the step of detecting that a gene which is operably linked to a second promoter and which encodes a second fluorescent protein exists in a target tissue, the second fluorescent protein is a protein that emits fluorescence with a color different from a color of fluorescence of the fluorescent protein constituting the fusion protein of the OLE1 and the fluorescent protein.

10. A method for producing a protein in a plant, comprising the steps of:
(a) inserting, to a DNA construct including a gene which is operably linked to an oleosin promoter consisting of a base sequence represented by SEQ ID NO: 6 and which encodes a fusion protein of OLE1 and a fluorescent protein, a second gene encoding a target protein; and
(b) introducing the DNA construct obtained in the step (a) to a plant,
the OLE1 consisting of an amino acid sequence represented by SEQ ID NO:1.

11. The method as set forth in claim 10, wherein the step (b) includes carrying out floral-dip or vacuum infiltration.

## Patentansprüche

1. DNA-Konstrukt, umfassend ein Gen, das für ein Fusionsprotein aus OLE1 und einem Fluoreszenzprotein kodiert, wobei das Gen operativ an einen Oleosin-Promoter verknüpft ist, der aus einer Basen-Sequenz besteht, die durch SEQ ID NO: 6 dargestellt ist,
wobei das OLE1 aus einer Aminosäuresequenz besteht, die durch SEQ ID NO: 1 dargestellt ist.

2. DNA-Konstrukt nach Anspruch 1, wobei
ein zweites, für ein Target-Protein kodierendes Gen und ein für ein zweites Fluoreszenzprotein kodierendes Gen operativ an den Oleosin-Promoter verknüpft sind, und
das zweite Fluoreszenzprotein ein Protein ist, das Fluoreszenz mit einer Farbe emittiert, die unterschiedlich von einer Fluoreszenzfarbe des Fluoreszenzproteins ist, das das Fusionsprotein aus dem OLE1 und dem Fluoreszenzprotein ausmacht.

3. DNA-Konstrukt nach Anspruch 1, weiter umfassend einen zweiten Promoter zum Exprimieren eines Target-Proteins in einem Target-Gewebe.

4. DNA-Konstrukt nach Anspruch 3, wobei
ein zweites, für ein Target-Protein kodierendes Gen und ein für ein zweites Fluoreszenzprotein kodierendes Gen operativ an den zweiten Promoter verknüpft sind, und
das zweite Fluoreszenzprotein ein Protein ist, das Fluoreszenz mit einer Farbe emittiert, die unterschiedlich von einer Fluoreszenzfarbe des Fluoreszenzproteins ist, das das Fusionsprotein aus dem OLE1 und dem Fluoreszenzprotein ausmacht.

5. Verwendung eines DNA-Konstrukts nach einem der Ansprüche 1-4 für einen Selektionsmarker.

6. Transgene Pflanze, in die ein Gen eingeführt ist, das für ein Fusionsprotein aus OLE1 und dem Fluoreszenzprotein kodiert, wobei das Gen operativ an einen Oleosin-Promoter verknüpft ist, der aus einer Basen-Sequenz besteht, die durch SEQ ID NO: 6 dargestellt ist,
wobei das OLE1 aus einer Aminosäuresequenz besteht, die durch SEQ ID NO:1 dargestellt ist.

7. Verfahren zum Selektieren eines transgenen Saatguts, umfassend die Schritte von:
Applizieren von Agrobacterium, das ein DNA-Konstrukt nach Anspruch 1 umfasst, auf eine Blütenknospe, um eine Pflanze zu transformieren und eine transgene Pflanze herzustellen; und
Detektieren, dass ein Gen, das für ein Fusionsprotein aus OLE1 und einem Fluoreszenzprotein kodiert, in einem Saatgut existiert, wobei das Gen operativ an einen Oleosin-Promoter verknüpft ist, der aus einer Basen-Sequenz besteht, die durch SEQ ID NO: 6 dargestellt ist,
wobei der Schritt des Detektierens, Detektieren von Fluoreszenz des Fluoreszenzproteins aus einem Saatgut umfasst, und
das OLE1 aus einer Aminosäuresequenz besteht, die durch SEQ ID NO:1 dargestellt ist.

8. Verfahren nach Anspruch 7, weiter umfassend den Schritt des Detektierens, dass ein Gen, das operativ an den Oleosin-Promoter verknüpft ist und das für ein zweites Fluoreszenzprotein kodiert, in einem Saatgut existiert, wobei das zweite Fluoreszenzprotein ein Protein ist, das Fluoreszenz mit einer Farbe emittiert, die unterschiedlich von einer Fluoreszenzfarbe des Fluoreszenzproteins ist, das das Fusionsprotein aus dem OLE1 und dem Fluoreszenzprotein ausmacht.

9. Verfahren nach Anspruch 7, weiter umfassend den Schritt des Detektierens, dass ein Gen, das operativ an einen zweiten Promoter verknüpft ist und das für ein zweites Fluoreszenzprotein kodiert, in einem Target-Gewebe existiert, dass das zweite Fluoreszenzprotein ein Protein ist, das Fluoreszenz mit einer Farbe emittiert, die unterschiedlich von einer Fluoreszenzfarbe des Fluoreszenzproteins ist, das das Fusionsprotein aus dem OLE1 und dem Fluoreszenzprotein ausmacht.

10. Verfahren zum Herstellen eines Proteins in einer Pflanze, umfassend die Schritte von:
(a) Insertieren eines zweiten, für ein Target-Protein kodierendes Gen in ein DNA-Konstrukt, umfassend ein Gen, das operativ an einen Oleosin-Promoter verknüpft ist, der aus einer Basen-Sequenz besteht, die durch SEQ ID NO: 6 dargestellt ist, und das für ein Fusionsprotein aus OLE1 und einem Fluoreszenzprotein kodiert; und
(b) Einführen des DNA-Konstrukts, das im Schritt (a) erhalten wurde, in eine Pflanze,
wobei das OLE1 aus einer Aminosäuresequenz besteht, die durch SEQ ID NO:1 dargestellt ist.

11. Verfahren nach Anspruch 10, wobei der Schritt (b) Ausführen von Floral-Dip oder Vakuuminfiltrierung umfasst.

## Revendications

1. Construction d'ADN, comprenant un gène codant pour une protéine de fusion d'OLE1 et d'une protéine fluorescente, le gène étant lié de manière fonctionnelle à un promoteur d'oléosine consistant en une séquence de base représentée par SEQ ID N° : 6,
l'OLE1 consistant en une séquence d'acides aminés représentée par SEQ ID N° : 1.

2. Construction d'ADN selon la revendication 1, dans laquelle
un second gène codant pour une protéine cible et un gène codant pour une seconde protéine fluorescente sont liés de manière fonctionnelle au promoteur d'oléosine, et
la seconde protéine fluorescente est une protéine qui émet une fluorescence ayant une couleur différente d'une couleur de fluorescence de la protéine fluorescente constituant la protéine de fusion de l'OLE1 et de la protéine fluorescente.

3. Construction d'ADN selon la revendication 1, comprenant en outre un second promoteur pour exprimer une protéine cible dans un tissu cible.

4. Construction d'ADN selon la revendication 3, dans laquelle
un second gène codant pour une protéine cible et un gène codant pour une seconde protéine fluorescente sont liés de manière fonctionnelle au second promoteur, et
la seconde protéine fluorescente est une protéine qui émet une fluorescence ayant une couleur différente d'une couleur de fluorescence de la protéine fluorescente constituant la protéine de fusion de l'OLE1 et de la protéine fluorescente.

5. Utilisation d'une construction d'ADN selon l'une quelconque des revendications 1 à 4 pour un marqueur de sélection.

6. Plante transgénique, dans laquelle est introduit un gène codant pour une protéine de fusion d'OLE1 et d'une protéine fluorescente, le gène étant lié de manière fonctionnelle à un promoteur d'oléosine consistant en une séquence de base représentée par SEQ ID N° : 6,
l'OLE1 consistant en une séquence d'acides aminés représentée par SEQ ID N° : 1.

7. Procédé de sélection d'une semence transgénique, comprenant les étapes :
d'application d'Agrobacterium incluant une construction d'ADN selon la revendication 1 à un bouton floral de sorte à transformer une plante et à préparer une plante transgénique ; et
de détection du fait qu'un gène codant pour une protéine de fusion d'OLE1 et d'une protéine fluorescente existe dans une semence, le gène étant lié de manière fonctionnelle à un promoteur d'oléosine consistant en une séquence de base représentée par SEQ ID N° : 6,
l'étape de détection incluant la détection de fluorescence de la protéine fluorescente à partir d'une semence, et
l'OLE1 consistant en une séquence d'acides aminés représentée par SEQ ID N° : 1.

8. Procédé selon la revendication 7, comprenant en outre l'étape de détection du fait qu'un gène qui est lié de manière fonctionnelle au promoteur d'oléosine et qui code pour une seconde protéine fluorescente existe dans une semence, la seconde protéine fluorescente étant une protéine qui émet une fluorescence ayant une couleur différente d'une couleur de fluorescence de la protéine fluorescente constituant la protéine de fusion de l'OLE1 et de la protéine fluorescente.

9. Procédé selon la revendication 7, comprenant en outre l'étape de détection du fait qu'un gène qui est lié de manière fonctionnelle à un second promoteur et qui code pour une seconde protéine fluorescente existe dans un tissu cible, la seconde protéine fluorescente est une protéine qui émet une fluorescence ayant une couleur différente d'une couleur de fluorescence de la protéine fluorescente constituant la protéine de fusion de l'OLE1 et de la protéine fluorescente.

10. Procédé de production d'une protéine dans une plante, comprenant les étapes :
(a) d'insertion, dans une construction d'ADN incluant un gène qui est lié de manière fonctionnelle à un promoteur d'oléine consistant en une séquence de base représentée par SEQ ID N° : 6 et qui code pour une protéine de fusion d'OLE1 et d'une protéine fluorescente, un second gène codant pour une protéine cible ; et
(b) d'introduction de la construction d'ADN obtenue à l'étape (a) dans une plante,
l'OLE1 consistant en une séquence d'acides aminés représentée par SEQ ID N° : 1.

11. Procédé selon la revendication 10, dans lequel l'étape (b) comprend la mise en oeuvre d'un trempage floral ou d'une infiltration sous vide.


### SEQUENCE LISTING

<110> Kyoto University
<120> A novel selection marker and use thereof
<130> KJ0910
<150> JP2008-140083
   <151> 2008-05-28
<160> 31
<170> PatentIn Ver. 2.1
<210> 1
   <211> 173
   <212> PRT
   <213> Arabidopsis thaliana
<400> 1
   Met Ala Asp Thr Ala Arg Gly Thr His His Asp Ile Ile Gly Arg Asp
    1               5                  10                  15
   Gln Tyr Pro Met Met Gly Arg Asp Arg Asp Gln Tyr Gln Met Ser Gly
               20                  25                  30
   Arg Gly Ser Asp Tyr Ser Lys Ser Arg Gln Ile Ala Lys Ala Ala Thr
           35                  40                  45
   Ala Val Thr Ala Gly Gly Ser Leu Leu Val Leu Ser Ser Leu Thr Leu
       50                  55                  60
   Val Gly Thr Val Ile Ala Leu Thr Val Ala Thr Pro Leu Leu Val Ile
   65                  70                  75                  80
   Phe Ser Pro Ile Leu Val Pro Ala Leu Ile Thr Val Ala Leu Leu Ile
                   85                  90                  95
   Thr Gly Phe Leu Ser Ser Gly Gly Phe Gly Ile Ala Ala Ile Thr Val
               100                 105                 110
   Phe Ser Trp Ile Tyr Lys Tyr Ala Thr Gly Glu His Pro Gln Gly Ser
           115                 120                 125
   Asp Lys Leu Asp Ser Ala Arg Met Lys Leu Gly Ser Lys Ala Gln Asp
       130                 135                 140
   Leu Lys Asp Arg Ala Gln Tyr Tyr Gly Gln Gln His Thr Gly Gly Glu
   145                 150                 155                 160
   His Asp Arg Asp Arg Thr Arg Gly Gly Gln His Thr Thr
                   165                 170
<210> 2
   <211> 199
   <212> PRT
   <213> Arabidopsis thaliana
<400> 2
   Met Ala Asp Thr His Arg Val Asp Arg Thr Asp Arg His Phe Gln Phe
    1               5                  10                  15
   Gln Ser Pro Tyr Glu Gly Gly Arg Gly Gln Gly Gln Tyr Glu Gly Asp
               20                  25                  30
   Arg Gly Tyr Gly Gly Gly Gly Tyr Lys Ser Met Met Pro Glu Ser Gly
           35                  40                  45
   Pro Ser Ser Thr Gln Val Leu Ser Leu Leu Ile Gly Val Pro Val Val
       50                  55                  60
   Gly Ser Leu Leu Ala Leu Ala Gly Leu Leu Leu Ala Gly Ser Val Ile
   65                  70                  75                  80
   Gly Leu Met Val Ala Leu Pro Leu Phe Leu Leu Phe Ser Pro Val Ile
                   85                  90                  95
   Val Pro Ala Ala Leu Thr Ile Gly Leu Ala Met Thr Gly Phe Leu Ala
               100                 105                 110
   Ser Gly Met Phe Gly Leu Thr Gly Leu Ser Ser Ile Ser Trp Val Met
           115                 120                 125
   Asn Tyr Leu Arg Gly Thr Arg Arg Thr Val Pro Glu Gln Leu Glu Tyr
       130                 135                 140
   Ala Lys Arg Arg Met Ala Asp Ala Val Gly Tyr Ala Gly Gln Lys Gly
   145                 150                 155                 160
   Lys Glu Met Gly Gln His Val Gln Asn Lys Ala Gln Asp Val Lys Gln
                   165                 170                 175
   Tyr Asp Ile Ser Lys Pro His Asp Thr Thr Thr Lys Gly His Glu Thr
               180                 185                 190
   Gln Gly Arg Thr Thr Ala Ala
           195
<210> 3
   <211> 191
   <212> PRT
   <213> Arabidopsis thaliana
<400> 3
   Met Ala Asn Val Asp Arg Asp Arg Arg Val His Val Asp Arg Thr Asp
   1               5                   10                  15
   Lys Arg Val His Gln Pro Asn Tyr Glu Asp Asp Val Gly Phe Gly Gly
               20                  25                  30
   Tyr Gly Gly Tyr Gly Ala Gly Ser Asp Tyr Lys Ser Arg Gly Pro Ser
           35                  40                  45
   Thr Asn Gln Ile Leu Ala Leu Ile Ala Gly Val Pro Ile Gly Gly Thr
       50                  55                  60
   Leu Leu Thr Leu Ala Gly Leu Thr Leu Ala Gly Ser Val Ile Gly Leu
   65                  70                  75                  80
   Leu Val Ser Ile Pro Leu Phe Leu Leu Phe Ser Pro Val Ile Val Pro
                   85                  90                  95
   Ala Ala Leu Thr Ile Gly Leu Ala Val Thr Gly Ile Leu Ala Ser Gly
               100                 105                 110
   Leu Phe Gly Leu Thr Gly Leu Ser Ser Val Ser Trp Val Leu Asn Tyr
           115                 120                 125
   Leu Arg Gly Thr Ser Asp Thr Val Pro Glu Gln Leu Asp Tyr Ala Lys
       130                 135                 140
   Arg Arg Met Ala Asp Ala Val Gly Tyr Ala Gly Met Lys Gly Lys Glu
   145                 150                 155                 160
   Met Gly Gln Tyr Val Gln Asp Lys Ala His Glu Ala Arg Glu Thr Glu
                   165                 170                 175
   Phe Met Thr Glu Thr His Glu Pro Gly Lys Ala Arg Arg Gly Ser
               180                 185                 190
<210> 4
   <211> 183
   <212> PRT
   <213> Arabidopsis thaliana
<400> 4
   Met Ala Asp Val Arg Thr His Ser His Gln Leu Gln Val His Pro Gln
    1               5                  10                  15
   Arg Gln His Glu Gly Gly Ile Lys Val Leu Tyr Pro Gln Ser Gly Pro
               20                  25                  30
   Ser Ser Thr Gln Val Leu Ala Val Phe Val Gly Val Pro Ile Gly Gly
           35                  40                  45
   Thr Leu Leu Thr Ile Ala Gly Leu Thr Leu Ala Gly Ser Val Ile Gly
       50                  55                  60
   Leu Met Leu Ala Phe Pro Leu Phe Leu Ile Phe Ser Pro Val Ile Val
   65                  70                  75                  80
   Pro Ala Ala Phe Val Ile Gly Leu Ala Met Thr Gly Phe Leu Ala Ser
                   85                  90                  95
   Gly Ala Ile Gly Leu Thr Gly Leu Ser Ser Met Ser Trp Val Leu Asn
               100                 105                 110
   Tyr Ile Arg Arg Ala Gly Gln His Ile Pro Glu Glu Leu Glu Glu Ala
           115                 120                 125
   Lys His Arg Leu Ala Asp Met Ala Glu Tyr Val Gly Gln Arg Thr Lys
       130                 135                 140
   Asp Ala Gly Gln Thr Ile Glu Asp Lys Ala His Asp Val Arg Glu Ala
   145                 150                 155                 160
   Lys Thr Phe Asp Val Arg Asp Arg Asp Thr Thr Lys Gly Thr His Asn
                   165                 170                 175
   Val Arg Asp Thr Lys Thr Thr
               180
<210> 5
   <211> 236
   <212> PRT
   <213> Arabidopsis thaliana
<400> 5
   Met Ala Gly Glu Ala Glu Ala Leu Ala Thr Thr Ala Pro Leu Ala Pro
    1               5                  10                  15
   Val Thr Ser Gln Arg Lys Val Arg Asn Asp Leu Glu Glu Thr Leu Pro
               20                  25                  30
   Lys Pro Tyr Met Ala Arg Ala Leu Ala Ala Pro Asp Thr Glu His Pro
           35                  40                  45
   Asn Gly Thr Glu Gly His Asp Ser Lys Gly Met Ser Val Met Gln Gln
       50                  55                  60
   His Val Ala Phe Phe Asp Gln Asn Asp Asp Gly Ile Val Tyr Pro Trp
   65                  70                  75                      80
   Glu Thr Tyr Lys Gly Phe Arg Asp Leu Gly Phe Asn Pro Ile Ser Ser
                   85                  90                  95
   Ile Phe Trp Thr Leu Leu Ile Asn Leu Ala Phe Ser Tyr Val Thr Leu
               100                 105                 110
   Pro Ser Trp Val Pro Ser Pro Leu Leu Pro Val Tyr Ile Asp Asn Ile
           115                 120                 125
   His Lys Ala Lys His Gly Ser Asp Ser Ser Thr Tyr Asp Thr Glu Gly
       130                 135                 140
   Arg Tyr Val Pro Val Asn Leu Glu Asn Ile Phe Ser Lys Tyr Ala Leu
   145                 150                 155                 160
   Thr Val Lys Asp Lys Leu Ser Phe Lys Glu Val Trp Asn Val Thr Glu
                   165                 170                 175
   Gly Asn Arg Met Ala Ile Asp Pro Phe Gly Trp Leu Ser Asn Lys Val
               180                 185                 190
   Glu Trp Ile Leu Leu Tyr Ile Leu Ala Lys Asp Glu Asp Gly Phe Leu
           195                 200                 205
   Ser Lys Glu Ala Val Arg Gly Cys Phe Asp Gly Ser Leu Phe Glu Gln
       210                 215                 220
   Ile Ala Lys Glu Arg Ala Asn Ser Arg Lys Gln Asp
   225                 230                 235
<210> 6
   <211> 1443
   <212> DNA
   <213> Arabidopsis thaliana
<400> 6
   tacttagatc aacacataaa agttagtaag tgaagaacca caacaacaac actagattca 60
   tcttcaagtg tatgtaggta tagtaacatg aacaagaaca gactcaagta caagatcgca 120
   tacgaaaatg gaaatggcaa tgtcacttcc acataatcaa acacgaatcc tcatatcaac 180
   aaggcctgag attctaacta gctcataaca acttagccaa tagttacttg agactaccaa 240
   atgtatgtag aactaaagac taagggacag agagttcgtc taaacaggtg aatctagtcg 300
   ttgttatcta ataaacaatt cagccccaaa tgcagaacac acatagagct ctctattgat 360
   tcaaattacg atctgatact gataacgtct agatttttag ggttaaagca atcaatcacc 420
   tgacgattca aggtggttgg atcatgacga ttccagaaaa catcaagcaa gctctcaaag 480
   ctacactctt tgggatcata ctgaactcta acaacctcgt tatgtcccgt agtgccagta 540
   cagacatcct cgtaactcgg attgtgcacg atgccatggc tatacccaac ctcggtcttg 600
   gtcacaccag gaactctctg gtaagctagc tccactcccc agaaacaacc ggcgccaaat 660
   tgcgcgaatt gctgacctga agacggaaca tcatcgtcgg gtccttgggc gattgcggcg 720
   gaagatgggt cagcttgggc ttgaggacga gacccgaatc cgagtctgtt gaaaaggttg 780
   ttcattgggg atttgtatac ggagattggt cgtcgagagg tttgagggaa aggacaaatg 840
   ggtttggctc tggagaaaga gagtgcggct ttagagagag aattgagagg tttagagaga 900
   gatgcggcgg cgatgagcgg aggagagacg acgaggacct gcattatcaa agcagtgacg 960
   tggtgaaatt tggaactttt aagaggcaga tagatttatt atttgtatcc attttcttca 1020
   ttgttctaga atgtcgcgga acaaatttta aaactaaatc ctaaattttt ctaattttgt 1080
   tgccaatagt ggatatgtgg gccgtataga aggaatctat tgaaggccca aacccatact 1140
   gacgagccca aaggttcgtt ttgcgtttta tgtttcggtt cgatgccaac gccacattct 1200
   gagctaggca aaaaacaaac gtgtctttga atagactcct ctcgttaaca catgcagcgg 1260
   ctgcatggtg acgccattaa cacgtggcct acaattgcat gatgtctcca ttgacacgtg 1320
   acttctcgtc tcctttctta atatatctaa caaacactcc tacctcttcc aaaatatata 1380
   cacatctttt tgatcaatct ctcattcaaa atctcattct ctctagtaaa caagaacaaa 1440
   aaa                                                               1443
<210> 7
   <211> 727
   <212> DNA
   <213> Vitis vinifera
<400> 7
   cctatcccca gcccacttcc acgtacacac cccatgcaag tcctggactt cactataaat 60
   aactgccctt catccccctc ttcactcact catcatcgat ccctctctct cattacaaaa 120
   aaacgatggc gaagctctca attttcgcag ctactctcct cctcctccta gccatctcca 180
   acgccaccat ctaccaaacc accgtcatca ccagggatga tgggtccgaa tttgggcagt 240
   tccaggggag ccagagccag aggtgcaggc agcagataca aggccagcag ttccagcagt 300
   gcgaacggta cattaggcag caagcggagc aacagcaggg cgggcagggt gacgtactga 360
   ttttacgggg catccgaaac cagcaacaac aggaacagca atggctccgc cagtgctgcc 420
   aagcgttgca gaacatggat cagcaatgcc agtgtgaggg tctccgccag atagtgcaaa 480
   ggcagcaggg tcagggtcag ggtcagggtc agggtcaggg tcggggtcag ggtcagggtc 540
   agggtcaggg acagggtcag ggtcagagag agcagcagca ggagatgatg cagatagcac 600
   agaagctgcc ggaaaggtgc ggctccggac aagcctgcca gagcatgcaa gttgtctggt 660
   tctagggctt ttgcagcggt gttgataata aagtacagtc acttacggtg actggaaacg 720
   agtaaca                                                           727
<210> 8
   <211> 549
   <212> DNA
   <213> Avena sativa
<400> 8
   tctagttaca gtaacaactt gtggaacatt acaaaattga tgtttgctag taacttctag 60
   aacactacaa cacttgacat gtataaggaa tttgatgagt catggcctac taaagcaagt 120
   tatattacta ctcttatcta tcttaacagg ccacacaaga ttacaaacta agttctgtat 180
   cagccatgct tatctagttt atgcataaca atttgcagaa cattacaaac ttagtttcgg 240
   aaaaataggc aatctagatt agtgtttgag ctataaagtg aataagatga gtcatgcgtg 300
   ttaacacacc tctttggtgg tggaatgata gtgcaacaac atgaaacttt agtgactagt 360
   ccaagaatac acatgtaagt agtgccacca aacacaacat accaaattat gattttggga 420
   agcatccaag cactttccag acaagaaaat gccaattgtg aaagagatca tgccatggga 480
   gctataaaaa gccttgtagc atgatcatca tccttcctca cccatcattc tcataagtag 540
   agcgcatca                                                         549
<210> 9
   <211> 666
   <212> DNA
   <213> Soybean
<400> 9
   caaaaacgca atcacacaca gtggacccaa aagccatgca caacaacacg tactcaccaa 60
   ggtgcaatcg tgctgcccaa aaacattcac caactcaatc catgatgagc ccacacattt 120
   gttgtttgta accaaatctc aaacgcggtg ttctctttgg aaagcaacca tatcagcata 180
   tcacactatc tagtctcttg gatcatgcat gcgcaaccaa aagacaacac ataaagtatc 240
   ctttcgaaag caatgtccaa gtccatcaaa taaaattgag acaaaatgca acctcacccc 300
   acttcactat ccatggctga tcaagatcgc cgcgtccatg tgggtctaaa tgccatgcac 360
   atcaacacgt actcaacatg cagcccaaat tgctcaccat cgctcaacac atttcttgtt 420
   aatttctaag tacactgcct atgcgactct aacccgatca caaccatctt ccgtcacatc 480
   aattttgttc aattcaacac ccgtcaactt gcatgccacc ccatgcatgc aagttaacaa 540
   gagctatatc tcttctatga ctataaatac ccgcaatctc ggtccaggtt ttcatcatcg 600
   agaactagtt caatatccta gtatacctta ataaataatt taatatacta tgatgagagc 660
   gcggtt                                                            666
<210> 10
   <211> 1026
   <212> DNA
   <213> Cauliflower mosaic virus
<400> 10
   ctagagccaa gctgatctcc tttgccccgg agatcaccat ggacgacttt ctctatctct 60
   acgatctagg aagaaagttc gacggagaag gtgacgatac catgttcacc accgataatg 120
   agaagattag cctcttcaat ttcagaaaga atgctgaccc acagatggtt agagaggcct 180
   acgcggcagg tctcatcaag acgatctacc cgagtaataa tctccaggag atcaaatacc 240
   ttcccaagaa ggttaaagat gcagtcaaaa gattcaggac taactgcatc aagaacacag 300
   agaaagatat atttctcaag atcagaagta ctattccagt atggacgatt caaggcttgc 360
   ttcataaacc aaggcaagta atagagattg gagtctctaa gaaagtagtt cctactgaat 420
   caaaggccat ggagtcaaaa attcagatcg aggatctaac agaactcgcc gtgaagactg 480
   gcgaacagtt catacagagt cttttacgac tcaatgacaa gaagaaaatc ttcgtcaaca 540
   tggtggagca cgacactctc gtctactcca agaatatcaa agatacagtc tcagaagacc 600
   aaagggctat tgagactttt caacaaaggg taatatcggg aaacctcctc ggattccatt 660
   gcccagctat ctgtcacttc atcaaaagga cagtagaaaa ggaaggtggc acctacaaat 720
   gccatcattg cgataaagga aaggctatcg ttcaagatgc ctctgccgac agtggtccca 780
   aagatggacc cccacccacg aggagcatcg tggaaaaaga agacgttcca accacgtctt 840
   caaagcaagt ggattgatgt gatatctcca ctgacgtaag ggatgacgca caatcccact 900
   atccttcgca agacccttcc tctatataag gaagttcatt tcatttggag aggactccgg 960
   tatttttaca acaataccac aacaaaacaa acaacaaaca acattacaat ttactattct 1020
   agtcga                                                            1026
<210> 11
   <211> 16
   <212> PRT
   <213> Artificial Sequence
<220>
   <223> Description of Artificial Sequence:synthesized peptide
<400> 11
   Cys Val Thr Ser Gln Arg Lys Val Arg Asn Asp Leu Glu Glu Thr Leu
    1               5                  10                  15
<210> 12
   <211> 25
   <212> DNA
   <213> Artificial Sequence
<220>
   <223> Description of Artificial Sequence:primer
<400> 12
   caccctactt agatcaacac ataaa 25
<210> 13
   <211> 22
   <212> DNA
   <213> Artificial Sequence
<220>
   <223> Description of Artificial Sequence:primer
<400> 13
   gagtagtgtg ctggccacca cg 22
<210> 14
   <211> 26
   <212> DNA
   <213> Artificial Sequence
<220>
   <223> Description of Artificial Sequence:primer
<400> 14
   caccatggca ggagaggcag aggctt 26
<210> 15
   <211> 26
   <212> DNA
   <213> Artificial Sequence
<220>
   <223> Description of Artificial Sequence:primer
<400> 15
   ttagtcttgt ttgcgagaat tggccc 26
<210> 16
   <211> 30
   <212> DNA
   <213> Artificial Sequence
<220>
   <223> Description of Artificial Sequence:primer
<400> 16
   caccgggccc tacttagatc aacacataaa 30
<210> 17
   <211> 30
   <212> DNA
   <213> Artificial Sequence
<220>
   <223> Description of Artificial Sequence:primer
<400> 17
   gggccctcgc atgcctgcag gtcactggat 30
<210> 18
   <211> 30
   <212> DNA
   <213> Artificial Sequence
<220>
   <223> Description of Artificial Sequence:primer
<400> 18
   caccactagt tagtaagtga agaaccacaa 30
<210> 19
   <211> 29
   <212> DNA
   <213> Artificial Sequence
<220>
   <223> Description of Artificial Sequence:primer
<400> 19
   actagtcgca tgcctgcagg tcactggat 29
<210> 20
   <211> 30
   <212> DNA
   <213> Artificial Sequence
<220>
   <223> Description of Artificial Sequence:primer
<400> 20
   caccactagt gtatgtaggt atagtaacat 30
<210> 21
   <211> 30
   <212> DNA
   <213> Artificial Sequence
<220>
   <223> Description of Artificial Sequence:primer
<400> 21
   cagctcgctc atagtagtgt gctggccacc
<210> 22
   <211> 30
   <212> DNA
   <213> Artificial Sequence
<220>
   <223> Description of Artificial Sequence:primer
<400> 22
   cagcacacta ctatgagcga gctgattaag
<210> 23
   <211> 30
   <212> DNA
   <213> Artificial Sequence
<220>
   <223> Description of Artificial Sequence:primer
<400> 23
   tgtttgaacg attcacttgt gccccagttt
<210> 24
   <211> 30
   <212> DNA
   <213> Artificial Sequence
<220>
   <223> Description of Artificial Sequence:primer
<400> 24
   gggcacaagt gaatcgttca aacatttggc
<210> 25
   <211> 29
   <212> DNA
   <213> Artificial Sequence
<220>
   <223> Description of Artificial Sequence:primer
<400> 25
   actagtgatc tagtaacata gatgacacc
<210> 26
   <211> 30
   <212> DNA
   <213> Artificial Sequence
<220>
   <223> Description of Artificial Sequence:primer
<400> 26
   caccactagt gtatgtaggt atagtaacat
<210> 27
   <211> 29
   <212> DNA
   <213> Artificial Sequence
<220>
   <223> Description of Artificial Sequence:primer
<400> 27
   actagtgatc tagtaacata gatgacacc 29
<210> 28
   <211> 30
   <212> DNA
   <213> Artificial Sequence
<220>
   <223> Description of Artificial Sequence:primer
<400> 28
   caccaagctt caagtgtatg taggtatagt 30
<210> 29
   <211> 29
   <212> DNA
   <213> Artificial Sequence
<220>
   <223> Description of Artificial Sequence:primer
<400> 29
   aagcttgatc tagtaacata gatgacacc 29
<210> 30
   <211> 30
   <212> DNA
   <213> Artificial Sequence
<220>
   <223> Description of Artificial Sequence:primer
<400> 30
   caccgggccc ttcaagtgta tgtaggtata 30
<210> 31
   <211> 28
   <212> DNA
   <213> Artificial Sequence
<220>
   <223> Description of Artificial Sequence:primer
<400> 31
   gggcccatct agtaacatag atgacacc 28